Europäisches Patentamt

European Patent Office

Office européen des brevets

(11) Publication number: **0 113 162**
**B1**

# EUROPEAN PATENT SPECIFICATION

(45) Date of publication of patent specification: **19.07.89**

(21) Application number: **83305426.5**

(22) Date of filing: **15.09.83**

(51) Int. Cl.⁴: **A 61 K 45/02, A 61 K 31/70 //
(A61K45/02, 31:70)**

(54) **Anti-proliferative action of dsNRAs on tumor cells.**

(30) Priority: **16.09.82 US 418652**

(43) Date of publication of application:
**11.07.84 Bulletin 84/28**

(45) Publication of the grant of the patent:
**19.07.89 Bulletin 89/29**

(84) Designated Contracting States:
**CH DE FR GB IT LI**

(56) References cited:
**US-A-3 666 646
US-A-4 024 222
US-A-4 130 641**

(73) Proprietor: **HEM RESEARCH, INC.**
**12280 Wilkins Avenue
Rockville Maryland 20852 (US)**
(73) Proprietor: **THE JOHNS HOPKINS UNIVERSITY**
**3400 North Charles Street
Baltimore, Maryland 21218 (US)**

(72) Inventor: **Carter, William Alvin**
**1 Jaine Lane
Birchrunville Pennsylvania 19421 (US)**
Inventor: **Ts'O, Paul O.P.**
**2117 Folkstone Road
Lutherville Maryland 21093 (US)**

(74) Representative: **Smart, Peter John et al
W.H. BECK, GREENER & CO 7 Stone Buildings
Lincoln's Inn
London WC2A 3SZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa. England.

## Description

This invention relates to double strande ribose nucleic acid (hereinafter referred to as dsRNA) in combination with interferon (hereinafter referred to as IFN), for use to inhibit the proliferation of neoplastic cells in an animal body including humans, to correct defects present in tumorous or cancerous cells in an animal body, including humans, and to treat immune system disorders stemming from the predisposition of certain individuals to be susceptible to tumorous or cancerous conditions, whether said predisposition arises from either genetic (hereditary) or environmental causes. The invention further relates to the use of dsRNAs in combination with hemin to treat tumorous or cancerous conditions.

### I. IFN Functions and dsRNAs as IFN Inducers

IFNs constitute a class of proteins capable of inhibiting virus replication in animal cells. They are of cellular origin, may be produced *in vitro* or *in vivo*, and are stimulated by a fairly wide range of materials (The Encyclopedia of Biochemistry, edited by Roger Williams and Edwin Lansford, Reinhold Publishing Company, 1967). IFN is known to exert antiproliferative effects as well as antiviral effects (Nature, *262*, 300, 1976; Exptl. Cell Res., *121*, 111, 1979). IFN is further known to reduce plasminogen activator secretion (Nature, *276*, 828, 1978), to reestablish contact inhibition (J. Cell Biol., *56*, 846, 1973), and to inhibit the growth in semi-solid agar of neoplastic cells (Nature, *231*, 20, 1971).

dsRNAs are inducers of the different molecular forms of human IFN including (leukocyte), (fibroblast), and (immune) types (J. Reticuloendothelial Society, *23*, 299, 1978) with IFN production capable of being stimulated by both natural and synthetic dsRNAs. The use of synthetic dsRNA in the form of complexes of polyriboinosinic and polyribocytidilic acids (hereinafter referred to as $rI_n \cdot rC_n$) strictly as an inteferon inducer is known, for example, from U.S. Patent 3,666,646 issued to Lampson et al. In addition to its role of IFN induction, dsRNA is also an activator of two IFN-induced enzymes, a protein kinase and a 2'-5' oligoadenylate synthetase (Proc. natl. Acad. Sci., *75*, 5893, 1978). The molecular bases for the antiproliferative and antineoplastic effects of interferon and their relationship to dsRNA are, however, unknown, and prior to the present invention, any antiproliferative effects of dsRNA beyond its role as an interferon inducer were also unknown.

### II. Synthetic mismatched analogs of dsRNA

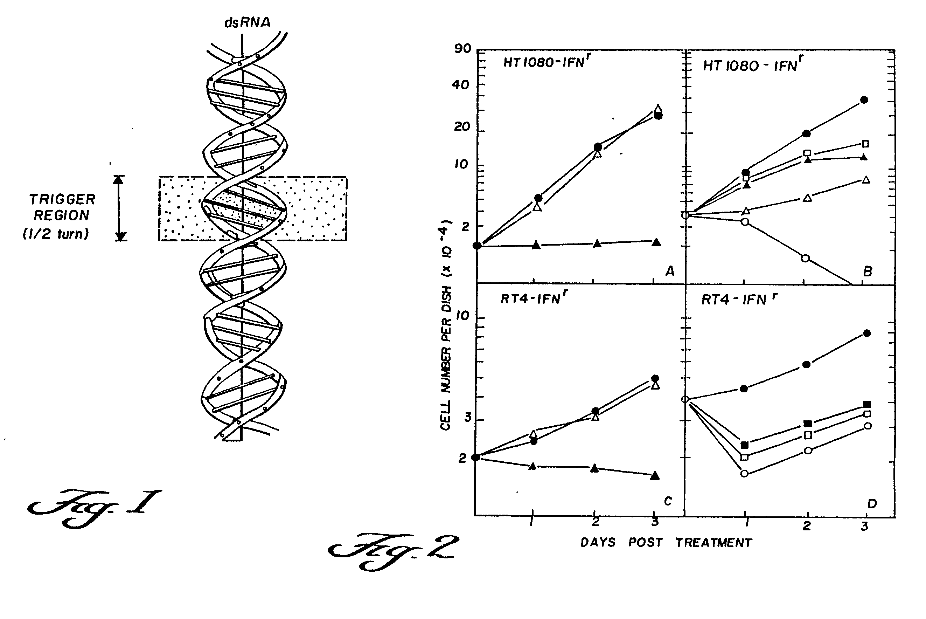

More recently, it has been discovered that IFN can also be induced by mismatched analogs of $rI_n \cdot rC_n$, as disclosed in U.S. Patents 4,130,641 and 4,024,222 issued to Ts'o and Carter, which patents are herein incorporated by reference. These analogs are formed by modifying $rI_n \cdot rC_n$ to incorporate unpaired bases (uracil or guanine) along the polyribocytidylate ($rC_n$) strand or by modifying the ribosyl backbone of polyriboinosinic acid ($rI_n$). The mismatched analogs, particularly those modified in the $rC_n$ strand, retain the ability to induce interferon so long as certain structural requirements are met, as shown in Figure 1. As Figure 1 illustrates, long regions of perfectly base paired dsRNA are not required for interferon induction. In fact, the structural prerequisite for triggering interferon synthesis appears to be the preservation of 1/2 to 1 helical turn of perfectly base paired dsRNA.

We have noted an accelerated rate of hydrolysis when the mismatched polymers were exposed to nucleases (J. Molec. Biol., *70*, 567, 1972), yet these dsRNA's were nearly as active as unmodified $rI_n \cdot rC_n$ in terms of interferon induction. It was proposed that prolonged maintenance of an intact double helical structure might be responsible for the many other biological responses to dsRNA commonly scored as drug toxicities. Studies of the mismatched analog, $rI_n \cdot r(C_{12},U)_n$ (herein also referred to as "Ampligen", a trademark of HEM Research, Inc., Rockville, Md. 20852) in various animal systems have shown $rI_n \cdot r(C_{12},U)_n$ to be less toxic than $rI_n \cdot rC_n$ (see Poly IC with mismatched bases, prospects for cancer therapy, in *Augmenting Agents in Cancer Therapy*, edited by E. M. Hersh et al., Raven Press, New York, 177, 1981). In particular, repeated administration of $rI_n \cdot r(C_{12},U)_n$ to mice results in much less toxicity to critically sensitive organs including bone marrow elements, liver cells, thymocytes and splenocytes (J. Molec. Biol., *70*, 567, 1972), while effectiveness in protecting against a variety of lethal viral challenges is preserved (Molec. Pharm., *12*, 299, 1976).

Additionally, $rI_n \cdot r(C_{12},U)_n$ exhibits reduced (100-fold reduction) pyrogenicity relative to $rI_n \cdot rC_n$ in rabbits, lower mitogenicity (5 to 10 fold reduction) with mouse and human cells *in vitro*, and decreased dsRNA antibody formation (5 to 10 fold reduction) in rabbits and mice. Against this background of reduced cellular toxicity, $rI_n \cdot r(C_{12},U)_n$ preserves its ability to induce interferon, stimulate natural killer (NK) cell function (J. Immuno., *124*, 1852, 1980), and activate the aforementioned intracellular mediators, 2'-5' · oligo-adenylate synthetase and a specific protein kinase (the co-inventors' unpublished data). It is therefore clear that, in a variety of test systems (rabbit, mouse, and man) and under different dose schedules measuring various toxicities and therapeutic effects, the mismatched analog, $rI_n \cdot r(C_{12},U)_n$ (Ampligen) has an enhanced therapeutic ratio. The preparation and formulation of this mismatched inducer/activator has been described (J. Molec. Bio. *70*, 567, 1972).

The use of modified $rI_n \cdot rC_n$ analogs for the sole purpose of inducing interferon production in animal cells to combat viral attack is known, for example, from the aforementioned U.S. Patents 4,130,641 and 4,024,222. However, with respect to its use as an antiproliferative agent, an impediment to IFN therapy exists in that IFNs exhibit only a marginal effect in many individuals. Consequently, relatively large amounts of IFN are required for administration, resulting in unnaturally high bodily IFN levels that would not be generated in the course of normal human response to an ordinary viral infection. At these high levels, the body can treat IFN as a foreign substance, and treated individuals have the capability to generate antibodies against IFN.

Moreover, neoplastic cells in particular have the capability to acquire resistance to the therapeutic properties of IFN. As it will be demonstrated infra, this ability of neoplastic cell lines to develop resistance against IFN is acquired as a non-random phenotype, and it therefore poses a severe drawback to any therapeutic method designed to inhibit proliferation predicated on the use of IFN alone. The present invention remedies this prior art deficiency by disclosing novel antiproliferative agents and compositions which halt or dramatically retard increases in cancerous cells, whether or not the cells have developed IFN resistance. Moreover, this invention provides compositions containing interferon wherein the bodily levels of interferon, although lower by a factor of 20 to 50 than the levels achieved by the prior art, result nonetheless in an even greater therapeutic effectiveness. Because the compositions of this invention containing dsRNA act synergistically, the levels of dsRNA required for administration are similarly lower than those disclosed by the prior art.

III. Natural killer (NK) cells as an immunosurveillance mechanism against tumor cells

Numerous papers have been published during the last two years which support a central role for NK cell populations in immunosurveillance mechanisms against neoplastic cells. It had been shown that mice with high NK-cell activity were more resistant to the growth of NK-sensitive tumor cells than were mice with low NK-cell activity (Immunol. Rev., *44*, 165, 1979). More recently, it was reported that C57BL/6 beige mice, which have a selective NK-cell defect, are more susceptible to tumor growth than are syngeneic mice with normal NK-cell activity (Naure (Lond.), *284*, 622, 1980; Nature, *284*, 624, 1980). A similar condition exists in humans with Chediak-Higashi syndrome and it has been suggested that the NK-cell defect in this disease may be causally related to the subsequent development of lymphomas in these patients (J. Exp. Med., *151*, 1039, 1980; J. Exp. Med., *151*, 1049, 1980; Nature (Lond.), *284*, 553, 1980). Patients with chronic lymphocytic leukemia (CLL) are known to have a high incidence of secondary malignancies. Recently, Ziegler and Zarling (Int. J. Cancer, *27*, 321, 1981) assessed NK cell activity in lymphocytes from chronic leukemia patients following removal of the leukemic cells. Significantly, the majority of CLL patients have minimal or no detectable NK cell activity despite the presence of lymphocytes which bind to NK-sensitive targets. In addition, renal allograft recipients, who are treated with high-dose immunosuppressive drugs, have an approximately 100-fold higher risk of developing malignancies and also have extremely depressed or abolished NK cell activities (Transplantation, *29*, 214, 1980). In contrast, antibody-dependent cell-mediated cytotoxicity (ADCC) is not depressed in these recipients. This seems to indicate that the defect (or lesion) in NK cell activity is a very specific one and, when present, is associated with a profound propensity for the development of malignancy.

Thus, recent evidence from several independent laboratories confirms the importance of the NK cell system as an immunosurveillance mechanism against neoplastic cells in man. We have now discovered that defects in human NK activity actually lead to human cancer and that this defect can be completely corrected with certain mismatched dsRNAs but not with interferon alone.

IV. IFN as the endogenous regulator of NK cell function

The principal usefulness of natural regulators such as interferon may be in the prevention of the overt malignant process by strengthening the 'physiologic control' of tumor clones, as they emerge, and strengthening pathways of natural immunity such as the NK cell system. We reason that without endogenous interferon, such pathways may be functioning below critical threshold levels or indeed may be altogether dormant. Again, recent evidence from several independent laboratories is confirming the role of interferon as the endogenous activator of the NK cell system. Hanna and Fidler (J. Nat. Cancer Inst., *64*(4), 80, 1980) have shown that interferon inducer treatment of three week old mice with low NK cell activity can boost NK cell activity and inhibit metastasis (tumor spread) formation. Reid et al. (Proc. Natl. Acad. Sci., USA, *78*(2), 1171, 1981) treated nude mice with antimouse interferon antiserum to reduce both interferon production and NK cell activity. Moreover, the anti-interferon treatment greatly increased the success rate of human tumor implantation and growth in the nude mice. The results are consistent with an important role for normal levels of endogenous interferon in restricting the growth, invasiveness, and metastases of tumor cells by acting indirectly through components of the immune system, such as NK cells. Finally, Edwards and Borden (Proceedings AACR, 1153, 1981) have shown that the NK cell activity of normal humans can be diminished or abrogated by a preincubation of the NK cells with anti-interferon antiserum. Thus, it appears that the continuous presence of cell surface bound interferon is critical for normal NK cell function in humans. Thus, recent evidence from many laboratories is confirming the intimate role of interferon as an endogenous regulator of the natural killer cell system. A brief overview of the endogenous induction of IFN is given by Table 1.

TABLE 1
Induction of human interferons

| Interferon type | Inducer | Comments |
|---|---|---|
| Fibroblast (beta, β) | $rI_n \cdot rC_n$ ConA | Production enhanced by inclusion of cycloheximide and Actinomycin D in induction scheme. |
| Leukocyte (alpha, α) | Sendai virus Newcastle disease virus | |
| Immune | PHA, ConA | Production enhanced by inducing lymphocytes in presence of macrophages. |
| Immune | Specific antigen | Lymphocytes must be sensitized to specific antigen. |
| Lymphoblastoid (Namalva) | Sendai virus | Human lymphoid cells containing an oncogenic virus. Cells produce primarily alpha interferon and small amounts of beta interferon. |

Our assays can measure minute quantities of interferon approximately 1 picogram/ml corresponding to a concentration of less than $1 \times 10^{-14}$ M.

Often the same cell type produces multiple interferon forms, indicating the existence of multiple genetic loci.

V. Augmentation of NK cell activity with exogenously applied interferon or the mismatched dsRNA.

We have observed that both purified human interferon and its inducers markedly augment the cytotoxic activity of human NK cells against a human leukemia cell line (K562). Also we noted that interferon activates normal lymphocytes to lyse fresh leukemic cells from many patients (J. Immunol., 124(4), 1852, 1980; J. Immunol., 123, 63, 1979). Furthermore, Hudlestone, Merigan and Oldstone (Nature, 282, 417, 1979) have suggested that defects in NK activity do occur in patients with lymphoma and their work suggests that exogenous interferon can correct these defects with a concomitant therapeutic effect.

We have found that dsRNA's exert antiproliferative effects beyond that inherent in their ability to induce IFN production. This capability is most strikingly proven by the fact that $rI_n \cdot rC_n$ or modified analogs thereof can inhibit the proliferation of IFN-resistant (hereinafter referred to as IFN$^r$) human tumor cells, i.e. tumor cells which have aquired the capability to grow and proliferate even in the presence of high concentrations of IFN. We have further determined, moreover, that even though tumor cells may be IFN-resistant, the use of exogenous IFN in combination with dsRNA results in a synergistic or potentiated effect with respect to that antiproliferative action. That is, the use of exogenous IFN and dsRNA in combination in individuals who exhibit only marginal response to IFN therapy results in an enhanced antiproliferative effect of dsRNA on neoplastic cells beyond that which results from therapy involving the use of dsRNA alone.

Additionally, for many individuals IFN therapy constitutes a feasible method of treatment. That is, these individuals do not require abnormally large dosage levels of IFN and, consequently, they run a much smaller risk of developing antibodies against IFN or sustaining other side-effects of the aministered IFN, said side-effects being related directly to IFN dosage. In these subjects, dsRNAs potentiate the action of IFN and thereby render IFN therapy an even more efficacious therapeutic method. Therefore, our therapeutic compositions improve existing treatment methods based of IFN alone and provide access to these treatment methods to individuals who would otherwise be precluded from efficacious treatment for one or more of the aforementioned reasons.

The invention provides a therapeutic composition comprising a therapeutic mixture of a dsRNA and an interferon.

Preferably the dsRNA is a mismatched analog of $rI_n \cdot rC_n$. As previously mentioned these mismatched analogs result from the interruption of either strand with unpaired bases (i.e. that do not form Watson-Crick base pairs) such as uracil or guanine. The analogs represent preferred embodiments due to the fact that they retain the antiproliferative effects of the unmodified $rI_n \cdot rC_n$ while displaying a greatly reduced tendency to trigger undesirable side effects such as fever, non-specific cell death (that is, the death of normal body cells), and antigenicity.

Moreover, we have also discovered that the mismatched analog, $rl_n \cdot r(C_{12},U)_n$ (Ampligen) exhibits dramatic effects even beyond those noted above in connection with the antiproliferative capabilities of a typical dsRNA such as $rl_n \cdot rC_n$. Specifically, it allows for the capability to correct defects in tumor cells such that they are reprogrammed and functionally converted to normal. We have in fact isolated cancer cells from patients and repeatedly observed this phenomenon. In addition, we have discovered that Ampligen can effectively function as a cancer cell normalizing agent in situations where IFN actually poses a complete obstacle to said treatment. Certain chemical and biological substances are known to possess some ability to independently normalize cancer cells. We have discovered and will demonstrate that Ampligen is able to amplify this type of tumor cell normalizing activity, but that IFNs often have the opposite effect, i.e., they reduce or nullify said normalization process.

Moreover, the present invention further allows for the correction of immune disorders in individuals predisposed to developing cancer by virtue of family background (i.e. heredity) or defects in the immune system. This capability is a non-IFN effect, and evidences the fact that mismatched dsRNA's can affect cancerous cells in a qualitative way that IFN alone does not.

Our therapeutic compositions may be used in the treatment of cancer, tumors, and neoplastic cells. "Treatment", in this context, is a generic term encompassing the prevention of cancer, tumor arrest (whether partial or complete), the failure of tumors to reappear in patients previously treated by established therapy, the conversion of tumor cells to normal, and the correction of cancer-related immune defects. Importantly, it is crucial to understand that this invention applies not only to the treatment of cancer or tumors once they have become clinically manifest, but also to the prevention or abortion of these malignancies while they are incipient or subclinical. That is, cancerous conditions may be subclinical for as long as decades before becoming overtly apparent and thereby becoming subject to existing therapeutic methods. Compositions according to the invention, on the other hand, provide for averting cancer before it ever reaches a clinically apparent stage, for example, in individuals having backgrounds indicating a high predisposition to cancer. Viewed from a different perspective, cancer is simply part of a (potentially decades long) biological continuum wherein the early stages of the continuum may be simply a predeposition. Our invention provides not only for the correction of overtly apparent cancer, but also allows for the prevention of cancer if its is treated during the early stages of this continuum, possibly decades before it actually appears.

It is therefore an object of this invention to provide therapeutic compositions useful for the treatment of cancer in animals, including humans, "treatment" (when used in conjunction with cancer, tumor, etc.) being a generic term encompassing the prevention of cancer, tumor arrest (whether partial or complete), the failure of tumors to reappear in patients previously treated by established therapy, the conversion of tumor cells to normal, or the correction of cancer related immune defects.

The invention includes the use of a dsRNA hemin and/or an interferon in the manufacture of a medicament for use in the treatment of cancer in either of the following combinations, namely (1) dsRNA and interferon and (2) dsRNA and hemin.

The invention will be illustrated and further described with reference being made to the accompanying drawings in which:—

Figure 1 is an illustration which shows disgramatically the structural requirement of dsRNA for IFN induction.

Figure 2 is a series of graphs illustrating, for two neoplastic cell lines, the heritability of resistance to the growth inhibitory effects of IFN, and the effect of $rl_n \cdot rC_n$ on the growth of IFNr cells in culture.

Figure 3 is a series of graphs showing, for two neoplastic cell lines, the effects of IFN on IFN$^s$ cells, and the effects of $rl_n \cdot rC_n$ on both IFN$^s$ and iFN$^r$ cells.

Fig. 4 is a graph showing the levels of SGOT, bilirubin, and various phosphatases following Ampligen treatment of a patient with prostatic carcinoma.

Fig. 5 is a tabulation which shows the progressive differentiation, during the first cycle of therapy with Ampligen, of cancer cells in a patient with chronic myelogenous leukemia.

Fig. 6 is a tabulation which shows the progressive differentiation, during the second cycle of therapy with Ampligen, of cancer cells in the patient (of Figure 5) with chronic myelogenous leukemia.

Fig. 7 is a photomicrograph of a cytocentrifuge preparation of leukemic myeloblasts on day 0 (Wright-Giemsa stain), which myeloblasts were isolated from peripheral blood following Ampligen therapy.

Fig. 8 is a photomicrograph similar that of Fig. 7, but showing a preparation of leukemic myeloblasts containing extensive cytoplasmic granulations after 7 days in culture.

Fig. 9 is a graph showing NK cell activity of normal individuals without cancer with either low or high family histories of cancer.

Fig. 10 is a graph showing the results of Fig. 9 separately for males and females.

Fig. 11 is a graph showing the results of Fig. 10 separately by age decade.

Fig. 12 is a series of graphs showing natural -IFN augmentation of NK cell activity in different types of individuals, showing in particular the ability of natural IFN, administered alone, to correct only partially the immune defects of humans predisposed to the development of cancer.

**I. IFN Resistance acquired as a non-random phenotype**

As previously stated, many individuals do not respond satisfactorily to cancer therapy based on the use of IFN alone. Consequently, high dosage levels are required with the result that such individuals are actually able to generate resistance to their natural bodily product, IFN. Moreover, the fact that resistance of another kind can also be developed by neoplastic cells as a non-randomly acquired phenotype is a consideration of paramount importance in understanding the dramatic improvement which the therapeutic compositions and methods of the present invention provide relative to any method which seeks to inhibit proliferation through the use of IFN alone. To demonstrate that human neoplastic cells can easily become resistant to the antiproliferative effects of IFN, we have characterised said effects on the human HT1080 fibrosarcoma and human RT4 epidermal carcinoma cell lines through the use of Luria-Delbruck fluctuation analysis (Genetics *28*, 491—511, (1943)), which indicates that the non-random acquisition of IFN-resistance as a phenotype is most likely induced by IFN at the time of selection.

The Luria-Delbruck fluctuation analysis, orginally developed for microbial systems, provides a statistical method whereby acquisition of a certain phenotype within a population of human cells can be determined to be a random (spontaneous) or non-random (induced) process. When a series of separate cultures, all initiated from a single clone, is allowed to undergo a suitable number of population doublings, the variation of the frequency of resistant colonies obtained in each culture will be large if the resistant trait is randomly acquired, or small if the trait non-randomly acquired. From a theoretical standpoint, the variance-to-mean ratio

$$\left( VMR, \ \frac{V1 - V2}{mean} \right)$$

will be less than or equal to one if the trait is acquired non-randomly, or much greater than one if the trait is acquired randomly.

To demonstrate the acquisition of IFN resistance as a non-random (induced) process, clonal populations of IFN-sensitive (hereinafter referred to as IFN[s]) cells were isolated from both the HT1080 and RT4 cell lines. The HT1080 clonal line (HT1080-2) and RT4 clonal line (RT4-1) demonstrated complete growth inhibition at 400 IU/ml and 80 IU/ml human fibroblast interferon (HuIFN-β) respectively, when examined over a 12-hour period. The fluctuation analysis was initiated by establishing 10 independent cultures from each original colony and continuously growing each culture for 16 population doublings, as described following Table 2, prior to plating in selective medium. The results are shown in Table 2, immediately below.

## TABLE 2
Luria-Delbruck fluctuation analysis for the acquisition of interferon resistance

| | HT1080 Human tumor | RT4 Human tumor | |
| --- | --- | --- | --- |
| Culture | IFN colonies per $10^3$ cells | 6TG$^r$ colonies per $10^6$ cells | IFN$^r$ colonies per $10^5$ cells |
| 1 | 39.3 | 7.6 | 126 |
| 2 | 36.9 | 2.4 | 145 |
| 3 | 38.2 | 6.1 | 127 |
| 4 | 35.7 | 14.2 | 127 |
| 5 | 41.1 | 0.0 | 97 |
| 6 | 42.5 | 0.0 | 102 |
| 7 | 44.6 | 18.0 | 96 |
| 8 | 39.1 | 0.0 | 119 |
| 9 | 37.1 | 2.5 | 122 |
| 10 | 35.9 | 2.7 | 108 |
| Variation | 7.73 | 35.25 | 224.09 |
| Mean | 39.04 | 5.35 | 116.90 |
| Random sampling variation | 2.04 | 0.16 | 187.40 |
| Corrected variance/mean | 0.15 | 6.56 | 0.31 |

The data in Table 2 for the HT1080 line were gathered by cloning HT1080 cells in semi-solid agar as described by Laug et al (J. Natl. Cancer Inst. *54*, 173, 1975). A vigorously growing colony was isolated and transferred to a 35 mm dish containing 2 ml growth medium, disbursed by repeated pipetting and allowed to attach and grow. The resultant monolayer was detached from the plate by trypsinization and was transferred to a 75 cm$^2$ flask for serial propagation. Cells comprising the resultant confluent monolayer were detached by trypsinization and 10 independent cultures were initiated by inoculating 10.35 mm dishes at 100 cells per dish. On reaching confluence, the total number of cells from each culture was detached by trypsinization and transferred to a separate 75 cm$^2$ flask to maintain the 10 cultures independently. After a total of 16 population doublings from initial establishment of the independent cultures, confluent monolayers in 75 cm$^2$ flasks were detached by trypsinization and the frequency of IFN$^r$ cells in liquid medium containing HuIFN-β at a plating density of $1 \times 10^4$ cells per 100 mm dish. Fluctuation analysis for the acquisition of 6TG$^r$ was simultaneously performed using selective conditions described previously (Mutat. Res. *70*, 221, 1980).

To obtain the data given in Table 2 for the RT4 line, an RT4 clone was isolated by the agar overlay method described in Methods in Cell Biology, *6*, 209—281, (1973), transferred to a 35 mm dish containing 2 ml growth medium and then handled in identical fashion to the HT1080 clone. RT4 cells were plated at a density of $1 \times 10^5$ cells per 100 mm dish in IFN-containing medium.

In the case of both the HT1080 and RT4 lines, selective medium consisted of growth medium containing HuIFN- (HT1080, 1000 IU/ml; RT4, 150 IU/ml) which was replaced twice per week, or 6TG (3 μg/ml). Results shown are corrected for plating efficiency. Random sampling variance was determined by analysing the frequency distribution observed in a single large culture in the same way as for independent cultures. Correction for sampling error was performed by subtracting the random sampling variance from the variance of the independent cultures before division by the means. Human IFN-β (fibroblast-derived) used in these studies had specific activities of $2 \times 10^6$ IU/mg protein (obtained from HEM Research, Rockville, Maryland) to $2 \times 10^7$ IU/mg protein (Dr W. A. Carter) after purification by sequential concanavalin A-sepharose and phenyl-sepharose affinity chromatographies (J. Biol. Chem., *251*, 7620, 1976).

For the HT1080-2 population, within each individual culture, 3.7—4.5% of the cells were capable of colony formation in IFN-containing medium (1000 IU/ml). No difference in average colony size or cell morphology was observed between control and IFN-treated cells. The frequency distribution of the number of resistant colonies obtained from this series of sister cultures exhibited a variance (V1) of 7.73. The variance of the distribution of the number of colonies in a large single culture (V2, random sampling control) was found to be 2.04. After correction for the random sampling control, the VMR of the frequency distribution of the number of resistant colonies obtained from the series of cultures was 0.15. In contrast, when RT4-1 cells were similarly treated, IFN-resistant colonies arose at a frequency of 0.11%, 30- to 40-fold less than the frequency observed for HT1080-2 cells (Table 2). However, the VMR of the distribution obtained from the series of 10 sister cultures still was found to be low, equaling 0.31. Simultaneous examination of the HT1080-2 cultures for acquisition of 6-thioguanine resistance (6TGʳ), an acquired trait of known spontaneous origin, resulted in a corrected VMR of 6.79 (Table 2). The high VMR is indicative of a randomly acquired trait and indicates that 16 population doublings were sufficient to distinguish between randomly and non-randomly acquired phenotypes. Using the $p_o$ method of Luria and Delbruck, a mutation rate of $1.8 \times 10^{-8}$ mutants/cell/generation can be calculated from the values presented in Table 2 for the acquisition of 6TGʳ. This rate is consistent with known mutation rates obtained for various loci in other system, verifying the correctness of the experimental procedure and integrity of the results.

The acquisition of IFN resistance, when analyzed in this fashion, provided VMRs of less than one, indicating that interferon resistance is a phenotype that is not the result of a spontaneous event, such as mutation or chromosomal rearrangement, but rather is a phenotype that is induced by IFN treatment.

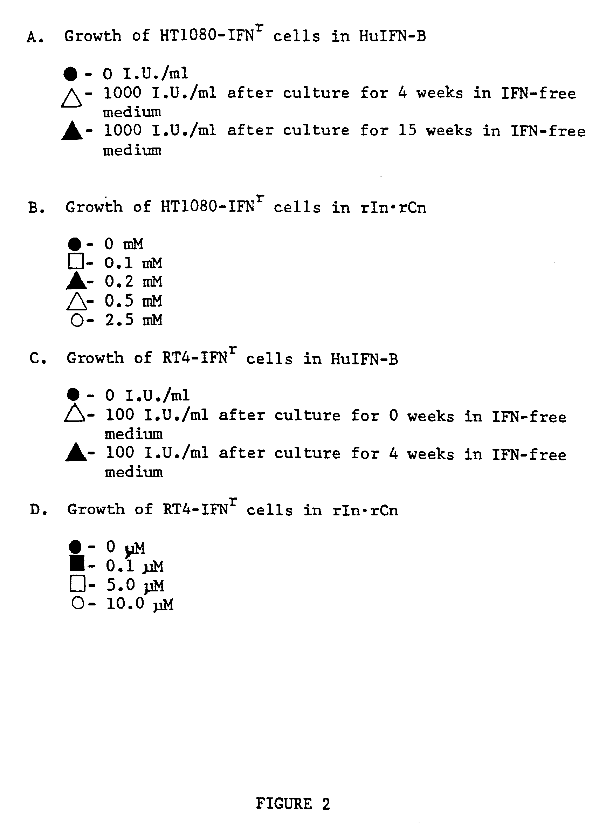

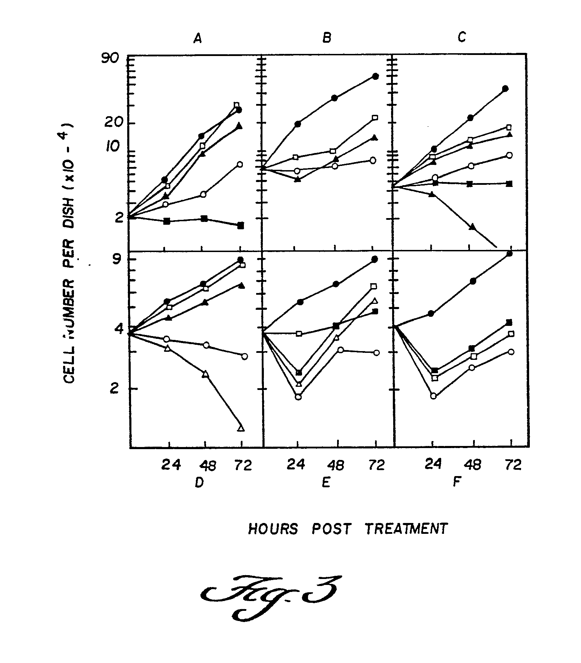

The acquisition of IFN-resistance as a non-randomly acquired phenotype was further characterized by examining the heritability of resistance to the growth inhibitory effects of IFN, the results for which are shown in Figures 2A and 2C. These figures were derived by examining colonies which were growing in IFN-containing medium and which were then isolated and grown in the presence of medium containing 1000 IU/ml (HT1080) or 100 IU/ml (RT4) HuIFN-β. After approximately 16 population doublings, HuIFN-β was removed from the culture medium and cells were examined for IFN-resistance at various passages after growth in IFN-free medium, as stipulated by the legend.

Examination of isolated colonies derived from resistant populations revealed IFNʳ cells possessed the same population doubling times in the presence and absence of IFN at concentration normally resulting in complete cessation of growth. However, consistent with the notion that IFN resistance is an induced trait, resistant colonies were found to revert to a sensitive state, albeit requiring varying lengths of time for reversion. One clone of HT1080-IFNʳ cells was found to retain the resistance trait after growth for four weeks in IFN-free medium, while reverting to a sensitive state by 15 weeks after removal of IFN from the culture medium (Figure 2A), whereas a clone of resistant RT4 cells was found to be fully resistant to IFN immediately after isolation, but reverted completely by four weeks after removal of IFN from the culture medium (Figure 2C). When employed as assay cells to titer an identical solution of IFN by the dye uptake method of Finter (N. J. Gen. Virol. 5, 419, 1969), resistant cells during their resistant state were found to be at least as sensitive as their parental populations to the antiviral effects of IFN when vesicular stomatitis virus was used as the challenge virus as shown by Table 3 immediately below.

TABLE 3

Sensitivity of parental IFNˢ and derived IFNʳ cells to the antiviral effects of IFN.

| Cell line | | Titer |
| --- | --- | --- |
| HT1080-cl 2 | IFNˢ | 1.5 |
| HT1080-cl 2 | IFNʳ | 4.5 |
| RT4-cl 1 | IFNˢ | 30.2 |
| RT4-cl 1 | IFNʳ | 28.0 |

Confluent monolayers (1.5 cm diameter) of IFNˢ or IFNʳ cells from both HT1080 and RT4 lines were used as assay cells for the titration of a stock solution of IFN (concentration 50 IU/ml), using the dye uptake method of Finter with vesicular stomatitis virus as the challenge virus.

The data in Table 3 indicate that resistance to the antiproliferative effects of IFN do not necessarily correlate with changes in sensitivity to the antiviral effects of IFN. However, it should be mentioned that different viruses can be affected by different mechanisms pertaining to the antiviral state (Nature 286, 178, 1980; Virol. 37, 827, 1981; Proc. Natl. Acad. Sci. 77, 2528, 1980).

The evidence provided by Figures 2A and 2C, simply stated, indicates that resistant cells previously sensitive to the growth inhibitory effects of IFN revert to a sensitive state, a conclusion supporting the proposition that IFN-resistance is a non-randomly acquired phenotype. Since no difference in average size was observed for colonies formed in control and IFN-β containing medium, induction of resistance must occur rapidly, within 24—48 hours after IFN exposure. Alternatively, cells within the population might exist in either an IFN$^r$ or IFN$^s$ state, depending on their physiological or developmental status. Addition of IFN would both select for the subpopulation of pre-existing resistant cells and also maintain the resistant trait.

The results of this phase of the invention contrast those of Gresser et al, who, by similar analysis, concluded that the resistance phenotype was randomly acquired and appeared at a frequency consistent with somatic mutation (J. Natl. Cancer Inst. 52, 553, 1974). However, Gresser et al obtained their high VMR from comparison of resistance displayed by different clones isolated from a mass tumor cell culture. Recent observations that clonal isolates from a population of tumor cells can vary widely in sensitivity to the antiproliferative effects of IFN (Proc. Natl. Acad. Sci. 77, 1471, 1980) indicate that such interclonal comparison cannot be used for determining the randomness of acquisition of IFN resistance. Moreover, resistant cells reported here, as opposed to those described by Gresser et al, retained their sensitivity to the antiviral effects of IFN, indicating that resistance was not due simply to the absence of an IFN receptor, and is consistent with previous reports where the antiviral state could be induced by IFN in Rous Sarcoma virus-transformed human fibroblasts resistant to the growth inhibitory effects of IFN (J. Gen. Virol. 33, 7, 1976).

## II. dsRNA's alone and in combination with IFN

In view of the participation of dsRNAs in the establishment of the antiviral state and the toxicity of exogenous dsRNA (Proc. Natl. Acad. Sci., 77, 2528, 1980), the IFN-resistant lines were characterized with regard to their sensitivities toward the antiproliferative effects of the synthetic dsRNA, $rl_n \cdot rC_n$ as shown in Figures 2B and 2D. To derive Figures 2B and 2D, which show the effects of $rl_n \cdot rC_n$ on the growth of IFN$^r$ cells (i.e. dsRNA-mediated toxicity), the same experimental procedure as described for Figures 2A and 2C was followed. Following the approximately 16 population doublings in the presence of HuIFN-$\beta$ at the concentrations noted, $5 \times 10^4$ (HT1080-IFN$^r$) or $1 \times 10^5$ (RT4-IFN$^4$) cells were plated per 35 mm dish and allowed to attach overnight. The following day (time 0), cell number per dish was determined, remaining monolayers were refed with various concentrations of $rl_n \cdot rC_n$ as noted by the legend and cell number per dish was determined every 24 hours thereafter.

Resistant lines of both HT1080-2 (Figure 2B) and RT4-1 (Figure 2D) were found to display identical sensitivity to $rl_n \cdot rC_n$-mediated toxicity as their respective parental HT1080-2 and RT4-1 lines. For HT1080-IFN$^r$ populations, 0.5—1.0 mM $rl_n \cdot rC_n$ was required to completely inhibit cell proliferation, with $rl_n \cdot rC_n$ concentrations in excess of 1 mM producing marked cell death within 24 hrs. Under these conditions, IFN concentrations secreted in the medium did not exceed 3 IU/ml. Detectable $rl_n \cdot rC_n$-induced toxicity in RT4-IFN$^r$ cells was observed at concentrations as low as 0.1 mM, with no induced IFN being detectable in the medium. However, in contrast to the prolonged cytotoxic effect observed in HT1080-2 cells, some clones of RT4-IFN$^r$ cells could recover from dsRNA-mediated toxicity after 24 hrs and exhibit the same growth rate as before treatment. This recovery was not the result of rapid degradation of $rl_n \cdot rC_n$, since re-addition of $rl_n \cdot rC_n$ to the culture medium at 24 or 48 hours did not alter the recovery growth rate. Addition of antisera, directed against human IFN-$\beta$ (final concentration 100 neutralizing units/ml), to the culture medium of $rl_n \cdot rC_n$-treated cells did not reduce or otherwise alter the extent of cytotoxicity induced by $rl_n \cdot rC_n$, indicating that low IFN concentrations did not play a role in $rl_n \cdot rC_n$-mediated toxicity.

Resistant cells were found to retain sensitivity toward dsRNA-induced toxicity that did not differ from that exhibited by the IFN$^s$ parental cell lines. This is an important consideration in the development of chemotherapeutic regimens for the treatment of cancer with IFN, and indicates that IFN used in combination with dsRNA would be much more effective in the treatment of cancer than IFN alone. In addition, the data argue against a role for the known IFN-induced, dsRNA-dependent enzymes in the antiproliferative effects of IFN. It has been shown that no detectable reduction in the extent of IFN-mediated growth inhibition was observed in mitogen-stimulated normal human fibroblasts pretreated with IFN for periods of up to 72 hrs and that continuous subculture of a sensitive line of normal human fibroblasts in the presence of HuIFN- for a period of up to six weeks does not result in the appearance of a resistant population, indicating that normal cells do not readily acquire IFN resistance.

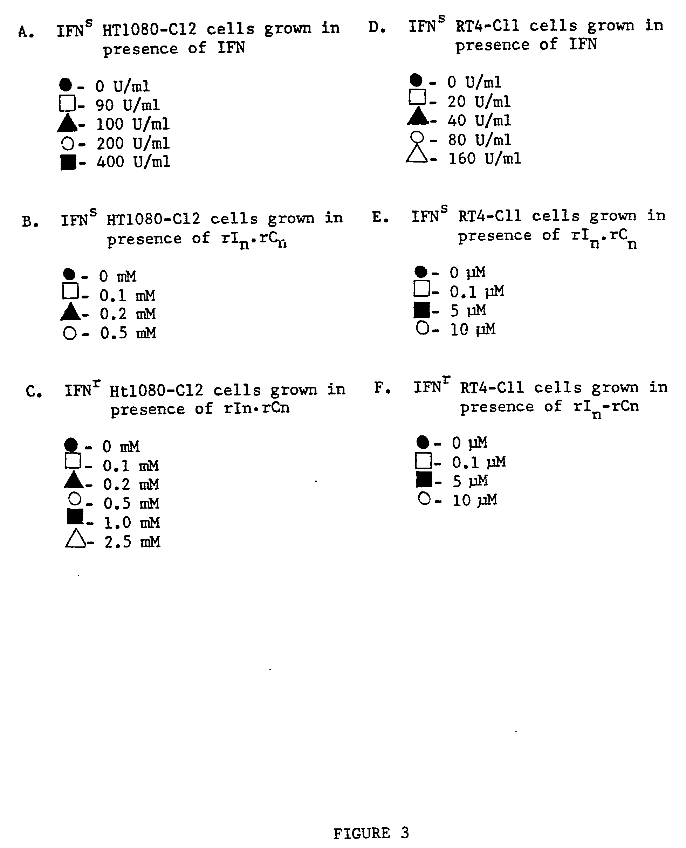

That dsRNAs such as $rl_n \cdot rC_n$ in fact exert a profound antiproliferative effect on human neoplastic cell lines may be seen from Figure 3. The data on which Figure 3 is based were gathered by growing HT1080 cells (Fig. 3, A—C) in the presence of Eagle's minimum essential medium supplemented with 10% fetal calf serum, and by growing RT4 cells (Fig. 3, D—F) in McCoy's medium supplemented with 15% fetal calf serum. Cells were plated at a density of $5 \times 10^4$ cells (HT1080) of $1 \times 10^5$ cells (RT4) per 35 mm dish in 2 ml growth medium and allowed to attach overnight. The following day (time 0), cell counts were obtained from duplicate dishes by trypsinization and counting with a hemacytometer. Either IFN or $rl_n \cdot rC_n$ was added to the remaining cultures and cell counts from duplicate plates were made every 24 hours thereafter over a 72-hour period. The concentrations of IFN or $rl_n \cdot rC_n$ are indicated in the legend to Figure 3. A comparison of the different subplots of Figure 3 clearly shows that $rl_n \cdot rC_n$ is capable of inhibiting the proliferation of IFN-sensitive (IFN$^s$) neoplastic cells on much the same order as IFN alone. The data further and importantly demonstrate that dsRNA can inhibit the proliferation of neoplastic cells that have become resistant to the antiproliferative effects of IFN alone.

Furthermore, Table 4, which is shown below, demonstrates that the antiproliferative action of $rl_n \cdot rC_n$ is not mediated by IFN since $rl_n \cdot rC_n$ can still exert an antiproliferative effect even in the presence of neutralizing amounts of antibody to IFN. Table 4 displays data derived by measuring the effects of anti-human IFN-β antibody on colony formation in the presence of $rI_n \cdot rC_n$ of $IFN^s$ and $IFN^r$ HT1080-2 and RT4-1 cells, and thereby provides a striking assessment of the reduction in cloning efficiencies of neoplastic cell lines due to the action of $rI_n \cdot rC_n$ alone. The data were gathered by inoculating HT1080 ($1 \times 10^3$) or RT4 ($5 \times 10^4$) cells into 100 mm dishes. The following day, medium containing $rI_n \cdot rC_n$ was added to each dish with or without anti-HuIFN-β antibody (final concentration when antibody added was 200 neutralizing units/ml). Colonies were allowed to form for 8 days (HT1080-c2) or 2 weeks (RT4-c1) prior to methanol fixation and Giemsa staining. The data clearly show that $rI_n \cdot rC_n$ precipitously reduces cloning efficiencies of $IFN^s$ or $IFN^r$ neoplastic cell lines, and that this effect occurs largely in the absence of any IFN influences on said cell lines.

TABLE 4

Effects of anti-HuIFN-β on cloning efficiencies of $IFN^s$ and $IFN^r$ lines
in the presence of $rI_n \cdot rC_n$.

| Cell line | IFN sensitivity | Antibody addition | $rI_n \cdot rC_n$ concentration | % Control |
|---|---|---|---|---|
| HT1080-cl 2 | $IFN^s$ | ± | 0 | 100 |
| | $IFN^s$ | − | $10^{-3}M$ | 3 |
| | $IFN^s$ | + | $10^{-3}M$ | 1 |
| HT1080-cl 2 | $IFN^r$ | ± | 0 | 100 |
| | $IFN^r$ | − | $10^{-3}M$ | 2 |
| | $IFN^r$ | + | $10^{-3}M$ | 2 |
| RT4-cl 1 | $IFN^s$ | ± | 0 | 100 |
| | $IFN^s$ | − | $10^{-5}M$ | 8 |
| | $IFN^s$ | + | $10^{-5}M$ | 7 |
| RT4-cl 1 | $IFN^r$ | ± | 0 | 100 |
| | $IFN^r$ | − | $10^{-5}M$ | 8 |
| | $IFN^r$ | + | $10^{-5}M$ | 7 |

In the case of cells which have developed IFN resistance, the antiproliferative action of $rI_n \cdot rC_n$ is potentiated by administration in combination with IFN, as demonstrated by the population doubling time data of Table 5 which is shown immediately below.

TABLE 5

Population doubling times of HT1080-$IFN^r$ at high IFN concentration

| $rI_n \cdot rC_n$ concentration (mM) | IFN concentration (U/ml) | | | |
|---|---|---|---|---|
| | 0 | 100 | 500 | 1000 |
| 0 | 17.7 | — | — | 16.6 |
| 0.5 | 49.3 | 73.1 | 96.9 | 142.1 |
| 1.0 | 133.8 | 139.1 | 143.1 | 142.1 |
| 2.5 | −42.7 | −42.9 | −33.0 | −33.7 |

The data were gathered by inoculating HT1080 cells ($1 \times 10^5$) into 35 mm cluster wells. After allowing 24 hours for attachment, the cultures were refed with medium containing IFN and/or $rI_n \cdot rC_n$ at varying concentrations. Population doubling times were calculated from cell counts determined every 24 hours for a 72-hour period following treatment. The negative numbers indicate time in hours for the number of cells to decrease by 1/2 (indicative of cell death). Because the cell lines were IFN-resistant, IFN alone would have little or no antiproliferative effect. It can therefore be seen from the data that the increased toxicity to neoplastic cells results from potentiated $rI_n \cdot rC_n$ action rather than from a mere additive IFN contribution to the antiproliferative effect.

The administration of IFN and any dsRNA, i.e. perfectly base-paired or mismatched, "in combination" includes embodiments wherein both agents are administered together as a therapeutic mixture, and also wherein both agents are administered separately but simultaneously, e.g., as through separate intravenous lines into the same individual. Administration "in combination" further includes the separate administration of the aforementioned drugs, wherein one of the drugs is given first and followed shortly thereafter by the second. All three of the said embodiments possess inherent advantages. The separate but simultaneous administration, for example, allows independent control of each agent, and thereby provides for therapeutic optimization on an individual patient basis. Separate administration, for example, allows for IFN alone to establish an effect on cells, however marginal, which effect is then amplified by the use of Ampligen. Preparation of IFN and dsRNA as a mixture, of course, provides for instant access to this therapeutic composition in cases where optimum levels have previously been established. The preceding comments apply to all cases presented in this application where one therapeutic agent is administered "in combination" with another.

Similarly, the therapeutic agents and compositions of this invention can be administered via any of the bodily routes customarily used and known in the medical arts. Intravenous administration has been mentioned, but other routes including intramuscular, intrathecal, intracranial, and intraperitoneal are also well within the scope of this disclosure.

When IFN and any dsRNA (including Ampligen) are administered in combination, the dsRNA may be administered in an amount which will result in a level from 1—1,000 µg dsRNA/ml in bodily fluid i.e. that solution of serum, salts, vitamins, etc. which circulates within the organism and bathes the tissues. For example, administration of a composition containing 10 mg of dsRNA to an individual weighing 160 lb (72 kg) would result in a dsRNA level of approximately 1 µg/ml. Similarly, the amount of IFN present when administering a dsRNA-IFN combination is that amount which would result in a level of 1—100,000 IRU/ml in bodily fluid. The manner of administration of the combination may be as previously described i.e. as a mixture, administration separately but simultaneously, or sequentially. The point here is that the administration be functionally synergistic, whatever physical embodiment it takes.

When any dsRNA (including Ampligen) is administered alone it may be administered in an amount which will result in a level from 1—1,000 µg/ml in bodily fluid.

III. The Pharmacologic and cellular effects of mismatched dsRNA's

As previously stated, a preferred form of the present invention occurs when the dsRNA employed to inhibit proliferation of neoplastic cells is a mismatched dsRNA such as $rI_n \cdot rI_n(C_{12},U)_n$ (Ampligen). We have found that mismatched dsRNA's cause milder pharmacologic effects relative to perfectly base paired dsRNA's.

A. Pharmacologic effects of mismatched dsRNA

We have conducted a clinical study which demonstrates the beneficial therapeutic effects obtainable by employing the Ampligen. Five patients with a variety of advanced malignant disorders including multiple myeloma (2), acute (1) and chronic (1) myelogenous leukemia, and prostatic carcinoma (1) were treated intravenously in a Phase I—II study of Ampligen following appropriate Human Research Committee approvals and informed patient consents. Clinical evidence of antitumor effects was observed in three of the patients. The only significant side effect was transient fever lasting 4 to 6 hours. The following examples detail the results of the study for each patient.

Example 1

The first patient was a 59 year old white female with an eight year history of IgG multiple myleloma. She had been treated previously with several standard chemotherapeutic agents including melphalan, prednisone, cyclophosphamide and BCNU. In addition, local radiotherapy had been given on several occasions to help control bone pain. Most recently her progressive disease was documented while receiving hexamethylmelamine. Skeletal x-rays showed typical multiple lytic lesions throughout all major bones including femura, humori, pelvis, and skull. All chemotherapeutic agents were stopped two months prior to treatment with Ampligen. Ten doses of Ampligen were given using a modified Fibonacci (dose-escalation) sequence. A total of 450 mg was given over six weeks. The patient tolerated the treatment well and no clinically significant toxicities were noted. Side effects included chills, transient fevers, a mild elevation (2-fold) of serum creatinine and alkaline phosphatase. Importantly, no abnormalities of coagulation, liver or bone marrow function were observed as have been noted with $rI_n \cdot rC_n$. Of clinical significance was a decrease in the 24-hour excretion rate of urinary kappa chains from 5—7 gm/24 hours prior to therapy to 1—2 gm/24 hours after 4—5 weeks of treatment. In addition, at multiple times during treatment, evidence was obtained for increased levels of the IFN-associated intracellular mediator 2'-5'-oligoadenylate synthetase as well as low levels of serum interferon. After six weeks of therapy, the patient developed a urinary tract infection with subsequent gram negative septicemia and died. This is a common medical complication observed in patients with cancer, especially multiple myeloma.

Example 2

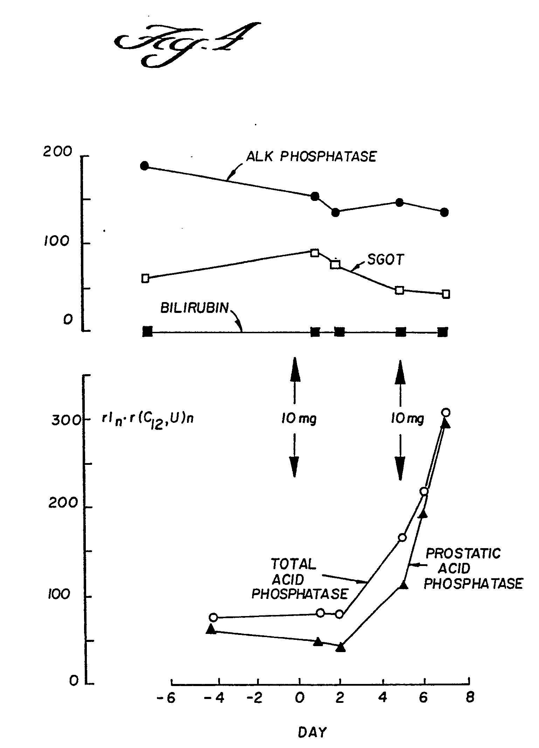

The second patient was a 66 year old white male with prostatic carcinoma metastatic to bone, liver and lungs. In addition, the patient had advanced heart disease and congestive heart failure. Following the first 10 mg dose of Ampligen, the patient noticed rapid relief from intense bone pain. Following the second 10 mg dose, the serum prostatic acid phosphatase rose six fold (Figure 4). There were no signs of liver, renal or bone marrow toxicities. The rapid increase in prostatic acid phosphatase probably indicates a specific effect of Ampligen on the patient's prostatic tumor cells. Other studies of the co-inventors in fact establish that Ampligen is unusually effective in preventing the multiplication of various cancers from the entire human genito-urinary tract.

Example 3

The third patient was a 48 year old white male with IgG multiple myeloma without Bence-Jones proteinuria. The patient received 70 mg of Ampligen over 3 weeks (10—15 mg doses twice weekly) with progression of his disease. No abnormalities of coagulation liver, renal, or bone marrow function were observed.

Example 4

The fourth patient was a 19 year old white male with acute myelogenous leukemia unresponsive to daunomycin, cytosine arabinoside and 6-thioguanine. The patient received three escalating doses of Ampligen (10, 15 and 30 mg) without any evidence of toxicity or response.

Example 5

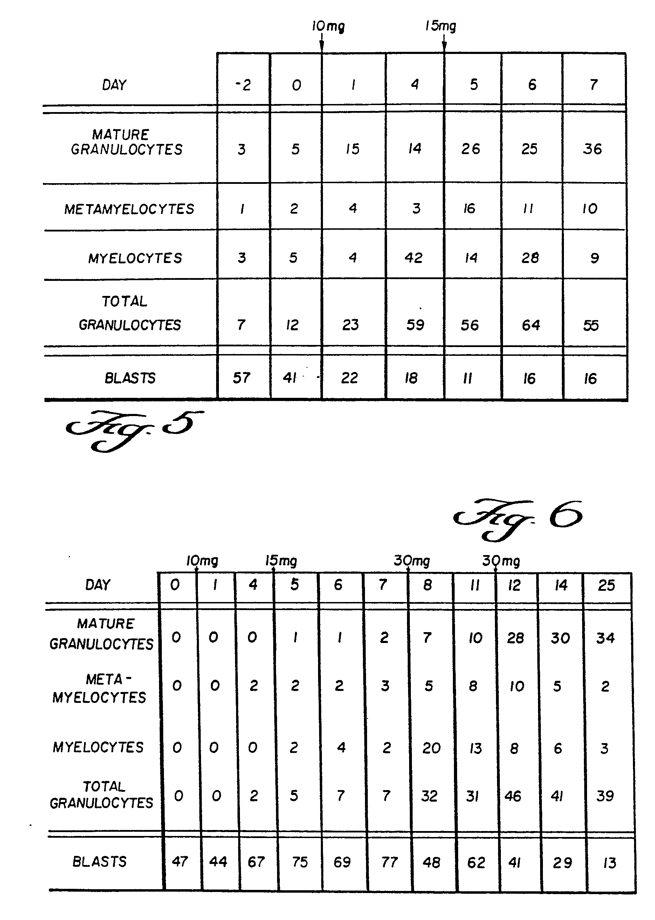

The fifth patient was a 45 year old white male with chronic myelogenous leukemia (Philadelphia chromosome positive) who entered blastic crisis after five years of conventional therapy for the chronic phase. Standard therapeutic approaches were exhausted when he failed to obtain a remission using a combination of m-AMSA plus 5-azacytidine, and Ampligen was then administered. The patient received two cycles of $rl_n \cdot r(C_{12},U)_n$ treatment separated by a three week rest interval. During both induction cycles, serial peripheral blood counts (Figures 5 and 6) were consistent with a differentiation promoting (i.e. cell normalizing) activity of Ampligen. Following induction cycle 2, the patient was actually placed on a maintenance regimen designed to control his white blood count while promoting differentiation (normalization) of a significant fraction of the leukemic cells in his body. The regimen consisted of Ampligen (10 mg every 1—2 weeks) plus hydroxyurea (1 gm) and 6-thioguanine (40 mg) daily. The latter two compounds *alone* had previously proven ineffective in controlling the disease. His disease became well controlled on this regimen for four months and he was able to resume his usual activities, including a work schedule of 10—12 hours/day. In addition, he maintained stable white blood cell counts between 14—22,000 per $mm^3$ with 32—58% mature granulocytes and 0.27% myeloblasts.

The clinical results show that Ampligen has *in vivo* activity against a variety of human tumors. Side effects were very minimal and included chills with transient fevers lasting 4—6 hours (4/5 patients), a very mild elevation of serum creatinine and alkaline phosphatase (both 2-fold in 1/5 patients). Most importantly, no abnormalities of coagulation (serum fibrinogen, fibrin split products, prothrombin, partial thromboplastin, and serial thrombin times), blood pressure (hypotension or hypertension), bone marrow function (leukopenia or thrombocytopenia), or renal liver function (except as noted above) were observed in any patient.

B. Cellular differentiating capabilities of mismatched dsRNA

In addition to the clinical studies exemplified above, we further conducted serial peripheral blood studies, the results for which are shown in the aforementioned Figures 5 and 6. The data affirmatively indicate that the administration of Ampligen actually allows for reprogramming of tumor cells, resulting in their functional conversion to normalcy. For Figure 5 (showing blood counts for the first cycle of therapy with Ampligen), the peripheral blood counts showed that the myelocyte peak (42%) preceded the metamyelocyte peak (16%), which in turn preceded the mature granulocyte peak (36%). Similarly, for Figure 6 (showing blood counts, for the second cycle of therapy with the Ampligen), the myelocyte peak (20%) preceded the metamyelocyte peak (10%), which in turn preceded the mature granulocyte peak (34%).

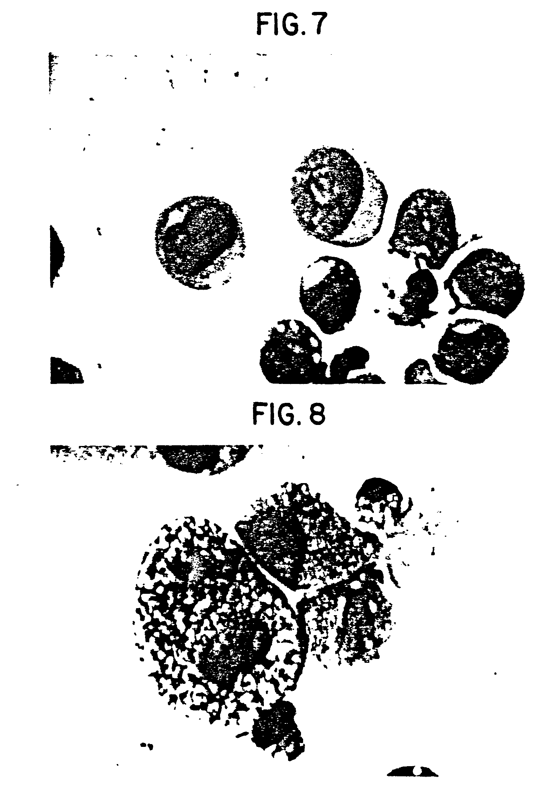

As concluded above, these studies indicate a differentiation promoting action of Ampligen allowing for reprogramming of tumor cells, and the ability of fresh peripheral blood myeloblasts to differentiate *in vitro* was therefore carefully examined. Normally, blastic cells from leukemic patients do not differentiate at all under our culture conditions. Accordingly, using Ficoll gradients (a method of purifying cells), myeloblasts were isolated from peripheral blood following Ampligen therapy and placed in short term culture to investigate the differentiation promoting action. Figure 7 shows the typical immature malignant nature of these fresh myeloblasts (day 0) immediately after being placed in culture. By day 3, eosinophilic granules, signaling the beginning of a differentiation process, appeared in the cytoplasms of 80—90% of the leukemic cells. By day 7, differentiation had progressed even further with the majority of cells now containing extensive cytoplasmic granulation (Figure 8). However, the majority of cells, *in vitro*, appeared blocked at promyelocyte/myelocyte level of differentiation with only a minority of cells progressing to the mature polymorphonuclear leukocyte or "band" stage. This result contrasts with the *in vivo* situation where the majority of blastic cells which began the differentiation program appeared to readily progress to mature cellular forms, i.e. they were completely normalized.

The results indicate that Ampligen demonstrates not only an antiproliferative effect against certain human tumor cells but can, in fact, also exhibit the novel property of actually promoting terminal differentiation of the tumor (leukemic) cell, i.e. the ability to convert or reprogram a tumor cell into a normal cell. The interferon system had already demonstrated in previous *in vitro* studies the ability to modulate cellular differentiation. Under some conditions interferon can inhibit the differentiation process (Proc. Natl. Acad. Sci. USA, *74*, 2036, 1977; Proc. Natl. Acad. Sci. USA, *77*, 4099, 1980), while in other situations interferon can promote cellular differentiation in lower animals (Cancer Res., *40*, 2919, 1980). In particular, Tomida and his co-workers have shown that mouse interferon can enhance the induction of differentiation of mouse myeloid leukemic (M) cells initiated by a group of apparently unrelated compounds. The differentiation promoting activity of Ampligen may be inherent to the dsRNA molecule itself, the biological mixture of interferons induced by $rl_n \cdot r(C_{12},U)$, or both. Whatever the mechanism involved, the ability to stimulate functional and morphological normalization of human tumor cells is a completely novel feature of dsRNA, Ampligen in particular, and one which we have invariably found cannot be readily observed when IFN alone is administered.

IV. Amplification of the effectiveness of various IFNs used to treat human tumors

As previously discussed, IFNs exhibit only a marginal anti-tumor effect in many individual and/or individual tumors such that therapy involving IFN alone can be impractical due to the high levels of IFN necessary for administration, with the attendant side-effects including antibody formation and other varied drug-related toxicities. By contrast, other individuals generate a much higher and more therapeutically meaningful response to IFN treatment, and it is these types of individuals for whom IFN anti-tumor treatment is most feasible. We have now discovered and herein demonstrate for such individuals that dsRNAs, including mismatched dsRNAs (Ampligen), amplify the effectiveness of the various kinds of IFNs used to treat human tumors even in situations wherein IFN alone exhibits a positive therapeutic response. The amplification of the IFN anti-tumor effect is a paramount consideration in developing individualized dosage regimens. As stated above, different individuals, and indeed, different tumors, exhibit different biological responses to IFN therapy. By combining dsRNAs, including Ampligen, with IFN, the variability in biological response across different individuals and/or tumors can be circumvented. In individuals for whom IFN treatment is a viable therapy, anti-tumor effects can be enhanced. Whereas, in the case of individuals whose response to IFN alone is altogether inadequate, combined IFN/dsRNA treatment makes feasible an anti-tumor therapy program which would otherwise be precluded.

To assess the amplification by dsRNAs of IFN anti-tumor effects, we formed a study group designed to show the effects of dsRNAs and IFNs in combination, and to separate out the effects of each of these agents alone. Said study group consisted of a control subgroup and 5 experimental subgroups as displayed by Table 6 following.

### TABLE 6

Amplification by dsRNAs of IFN anti-tumor effects against human bladder cancer

| | Subgroup | Treatment schedule | Tumor volume of 42 days as % of controls, ± standard error |
|---|---|---|---|
| I. | Control Albumin solution ("mock" treated mice) | daily | $100\pm12$ |
| II. | Human Fibroblast Interferon daily | $10^5$ international reference units (IRU) daily | $67\pm22$ |
| III. | Human Fibroblast Interferon plus $rl_n \cdot rC_n$ | $10^5$ IRU daily plus 100 mg 3 times per week | $45\pm7$ |
| IV. | $rl_n \cdot rC_n$ | 100 mg 3 times per week | $97\pm31$ |
| V. | $rl_n \cdot r(C_{12},U)_n$ [Ampligen] | 100 mg 3 times per week | $79\pm14$ |
| VI. | Human Fibroblast Interferon plus $rl_n \cdot r(C_{12},U)_n$ [Ampligen] | $10^5$ IRU daily plus 100 mg 3 times per week | $56\pm15$ |

Referring to Table 6, each subgroup consisted of 10 adult athymic mice which were injected on day zero with human bladder cancer cells (RT-4). The human tumor cells were injected subcutaneously onto the back region, thus allowing for accurate measurement of tumor growth by measuring tumor volume through the use of calipers. All drug injections were intraperitoneal.

Throughout the entire interval that tumors were palpable and measurable (day 13 to termination of experiment), the animals treated by combined IFN-dsRNA (including Ampligen) therapy showed the greatest suppression of human bladder tumor cell growth. Importantly, the combined antitumor effect of a dsRNA and IFN is synergistic as can be seen from a casual examination of the data in Table 6. That is, the combined effect is greater than that which would be expected by summing the contributions of IFN and dsRNA alone. Therefore said combined effect is a true amplification as opposed to being merely additive.

The activities of other IFN types besides human fibroblast IFN (subgroup II of Table 6) were also examined, including the natural varieties of human leukocyte IFN and IFNs produced in bacteria by use of recombinant DNA technology. In all cases, the IFNs demonstrated less effect on human tumor cell growth when injected alone than when injected together with or follow by a dsRNA, whether matched $(rI_n \cdot rC_n)$ or mismatched (Ampligen—$rI_n \cdot r(C_{12},U)_n$). The maximum reduction in human tumor cell growth achieved by any IFN administered alone was approximately 33% across a broad range of injected IFN concentrations ($10^5$ to $3 \times 10^6$ IRU daily). In contrast, animals treated with as little as $10^5$ IRU daily of IFN in combination with a dsRNA exhibited a 65% or greater reduction in human tumor cell growth. Accordingly, dsRNA used in conjunction with any single form of IFN results in a quantitatively superior effect. Moreover, human tumors generally require an individualized treatment regimen to generate the optimum therapeutic effect. We therefore believe that further efforts to individualize the treatment schedule will result in a 10-fold or greater therapeutic effectiveness whenever a mismatched dsRNA is used in conjunction with IFN.

The data clearly demonstrate that dsRNA will amplify the therapeutic effectiveness of all forms of IFN, including natural, synthetic (i.e. recombinant DNA-derived), and the hybrid forms such as those derived in part from alpha IFNs and in part from beta or gamma IFNs, and all are within the scope of this invention. Accordingly the combination of dsRNA and any IFN constitutes a potent formulation against cancer far exceeding the capability of IFN alone. Moreover, the human tumor material we studied is known to contain various types of viral genetic information which affirmatively contribute to the malignant process and/or the pathological processes in human tissue, causing tissue disease and destruction. The data therefore importantly demonstrate that a dsRNA-IFN combination is not only an effective anti-cancer agent, but also acts against viral components and diseases as well. Indeed, the therapeutic compositions of this invention will act against any disease in which IFN treatment may be indicated, including viral diseases manifested by either acute, subacute, latent, or chronic phase, and also including those dysimmune human diseases wherein viral activity serves to initiate the human disease pathology but may also disappear as the human disease pathology but may also disappear as the human "autoimmune" disease becomes self-perpetuating.

It should be emphasized that the role of any dsRNA in the synergistic enhancement of any combined dsRNA-IFN treatment is not as an IFN inducer, else no synergism would be observed. Rather, dsRNA is a uniquely contributing agent which supplements and complements the action of IFN, thereby providing a degree of flexibility and effectiveness which could not be attained by either dsRNA or IFN alone in the treatment of human viral diseases and cancer.

V. Ampligen versus IFNs in the conversion of human tumor cells to normalcy

In Section IV, supra, it has been shown that dsRNAs, including Ampligen, can work in concert with IFNs to synergistically inhibit the growth of human tumors. We herein now demonstrate that Ampligen can also cooperate synergistically with other chemical and biological substances to enhance the conversion of tumor cells to normal cells. These other substances themselves possess some capability to normalize human tumor cells, as does Ampligen. The combination of such substances together with Ampligen, however, produces a mutual potentiating effect. The important point we wish to emphasize is that if IFN also is combined with Ampligen and said substances, the net effect is diametrically opposed to synergism. That is, the addition of IFN acts to inhibit the beneficial enhanced anti-tumor effects of Ampligen in combination with other normalizing substances. The data (Table 7) will therefore demonstrate that Ampligen can affect neoplastic cells in a qualitative way that IFNs can not. Accordingly, we have provided an additional antiproliferative capability for the arsenal of anti-tumor compositions and methodologies already disclosed herein.

Leukemic cells are functionally "frozen" at a primitive stage in their development process, leading to the phenomenon of continuous tumor cell multiplication ("self-renewal"). Such uncontrolled multiplication is completely different from the normal pattern of orderly cell growth which terminates in a healthy individual when a certain critical concentration or configuration of normal cells has been reached. Moreover, malignancy not only causes this loss of the normal bodily biochemical constraints on cell number, but additionally circumvents the bodily constraints on cell location. For example, leukemic cells can migrate to the lungs, brain, viscera etc.

Ampligen used together with other agents can correct this underlying biochemical derangement. The data supporting this proposition are shown by Table 7, infra.

To obtain the data of Table 7, human leukemia cells (K562) were obtained from a patient with chronic mylogenous leukemia in blastic crisis. The cells were maintained in culture in RPMI 1640 medium (Gibco) supplemented with 10% fetal bovine serum (Gibco) and 1% penicillin/streptomycin (Gibco) at 37°C in 5% $CO_2$. For induction, the cells had been seeded at $4\text{---}5\times10^6$/ml in RPMI 1640 medium with 15% fetal bovine serum and 1% penicillin/streptomycin. Cultures were treated with 1 μg/ml of Ampligen, various concentrations of the different molecular varieties of IFN, and 0.05 mM Hemin (Bovine, Sigma). Induced cells were kept in culture for 96 hours. Daily cultures were counted for proliferation by trypan blue exclusion and tested for hemoglobin production by staining with benzidine using o-dianiside (Sigma).

The presence of hemoglobin is taken as evidence that the malignant hematopoietic cell (leukemia) has been reprogrammed to become a normal bodily constituent cell. Said presence was monitored after 24, 48, 72 and 96 hours of growth in the presence of hemin and Ampligen. Following each interval cells were pelleted, washed two times in Hank's BSS (Gibco) and cytospun onto slides. The slides were stained in 1% benzidine in methanol for 2 minutes, drained, placed in 2.5% $H_2O_2$ for 1 minute.

## TABLE 7
### Mismatched dsRNA activity

| Hours after addition of drugs | I<br>Control | II<br>Hemin 0.05 mM | III<br>$rI_n \cdot r(C_{12},U)_n$ (Ampligen) (1 μg/ml) plus Hemin (0.05 mM) |
|---|---|---|---|
| 0 | 0 | 0 | 0 |
| 24 hours | 0 | 28 | 38 |
| 48 hours | 0 | 62 | 72 |
| 72 hours | 0 | 68 | 74 |
| 96 hours | 0 | 54 | 79 |

| IV<br>Ampligen (1 g/ml) | V<br>Hemin (0.05 mM) plus Interferon* | VI<br>Ampligen (1 μg/ml) plus Interferon* plus Hemin (0.05 mM) |
|---|---|---|
| 0 | 0 | 0 |
| 0 | 24 | 16 |
| 0 | 22 | 39 |
| 0 | 15 | 11 |
| 3 | 23 | 20 |

Results are expressed as percentage of normal (reprogrammed tumor cells) cells in the culture

Hemin solution was prepared by dissolving 26 mg of hemin in 0.8 ml of 0.5 M NaOH and then neutralized with 1 ml of 1M Tris-HCl (pH=7.8—8.0). The solution was diluted to 10 mls with distilled water and filter sterilized. The concentration of hemin at this point was 4 mM. In Table 7, the concentration shown is 0.05 mM.

Referring to the data in Table 7, it has previously been well recognized that on exposure to a certain chemical substance termed hemin, a certain percentage of tumor cells can be converted to normal. Our experiments now establish a novel additional action by mismatched dsRNAs, namely the ability to assist other chemical or biological agents such as hemin in converting malignant cells to normal. The amplified hemin activity is clearly shown by a comparison of columns II and III. Moreover, the synergistic cooperation between the mismatched dsRNA, Ampligen, and hemin is vividly demonstrated by the fact that the concentration of Ampligen in these experiments was deliberately chosen so that Ampligen itself would have little if any normalizing effect (column IV). We have conducted like induction experiments using a variety of Ampligen levels including 100 μg, 10 μg, (Table 6), and 0.1 μg, as well as a variety of hemin concentrations (0.1 mM, 0.05 mM (Table 6), and 0.025 mM). In all cases results and trends in the data are comparable.

Comparison of columns II and V shows that IFN can actually inhibit the normalizing effect of hemin, an effect which contrasts the positive synergistic effect between Ampligen and hemin. Moreover, comparing columns III and VI shows that IFN can partially nullify the hemin-Ampligen cooperation. Whatever the normalization mechanism of hemin-Ampligen may be, said comparisons conclusively show not only that the mechanism is not the result of IFN induction, but also that said mechanism is actually at least partially blocked or abrogated by IFN. We have observed this negative IFN effect across a broad range of concentrations of IFN ranging between 5 to 2000 IRU/ml. Thus, we have now provided a method for normalizing tumor cells for situations wherein IFN has no effect or even constitutes a therapeutic hindrance.

Although our prototype example has been leukemia, our discovery extends to other tumorous or cancerous conditions as well wherein an Ampligen or other dsRNA can be used with Hemin to correct or reprogram human tumor cells to their normal cellular counterparts, including tumorous or cancerous conditions of the breasts, colon, prostate gland, etc., i.e. those conditions manifesting biochemical derangements similar to leukemia. By providing agents, compositions, and methods by which the malignant process can be prevented, corrected, aborted, or reprogrammed, our invention encompasses the capability to reverse or prevent human cancer in individuals whose cells have become pre-malignant, regardless of whether the malignant process is due to genetic predispositional, environmental, or other causes.

### V. Discovery of a new defect in human cancer

We have observed low levels of NK cell activity in some members of the study group including patients with lung cancer and individuals with a high family history of cancer. These observations are to be detailed below.

### A. Quantitative correction of the human NK defect by dsRNA working via a non-IFN mechanism.

We have tested whether low NK cell activity in patients with a high family history of cancer could be re-established to normal levels through the addition of various types of interferon and/or Ampligen. Recent reports demonstrate differences in the efficiency of NK cell augmentation (cells derived from normal individuals) among both natural types (Brit. J. Haem., *50*, 85, 1982) and cloned subtypes (Can. Res., *42*, 1312, 1982) of interferon. We observed that only mismatched dsRNA has the ability to reestablish normal levels of NK cell augmentation in individuals at risk to the development of cancer.

### B. Experimental demonstration of the NK defect in patients at risk to developing cancer.

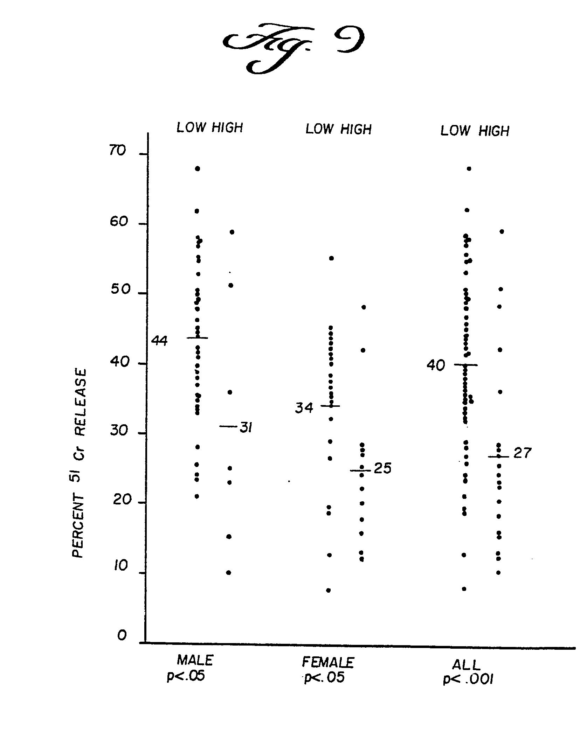

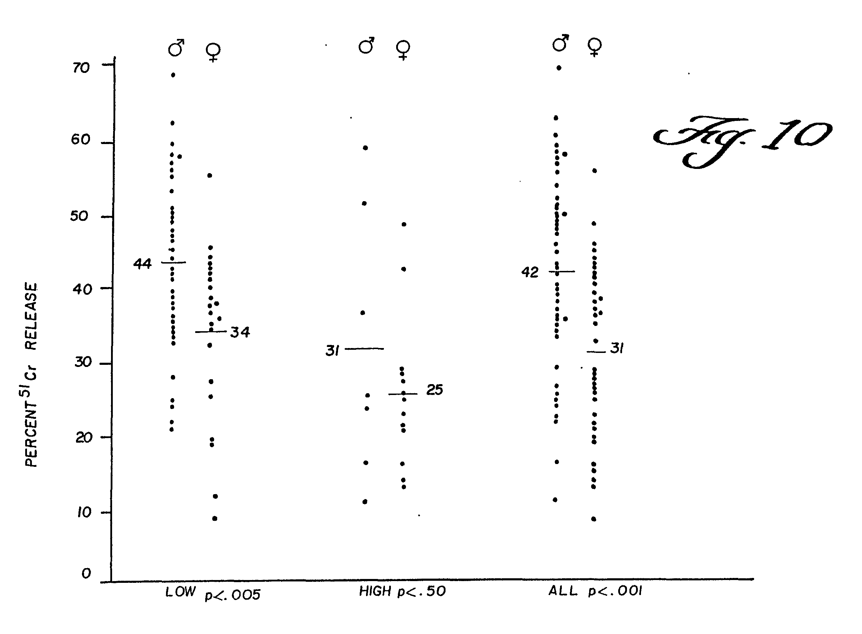

Figure 9, the results of which are shown separately for males and females, shows that individuals with a high family history of cancer have significantly lower ($p<.001$) NK cell activity (27.1% killing) than individuals with a low family history of cancer (40.0% killing). Figure 9 also shows that males with a low family history of cancer (43.5% killing) have higher NK cell activity ($p< .05$) than males with a high family history of cancer (31.3% killing). Similarly, females with a low family history of cancer (33.9% killing) have a higher NK cell activity ($p< .05$) than females with a high family history of cancer (24.8% killing). A high family history of cancer is defined as having three relatives with cancer (other than non-melanomatous skin cancer) among grandparents, parents, and siblings.

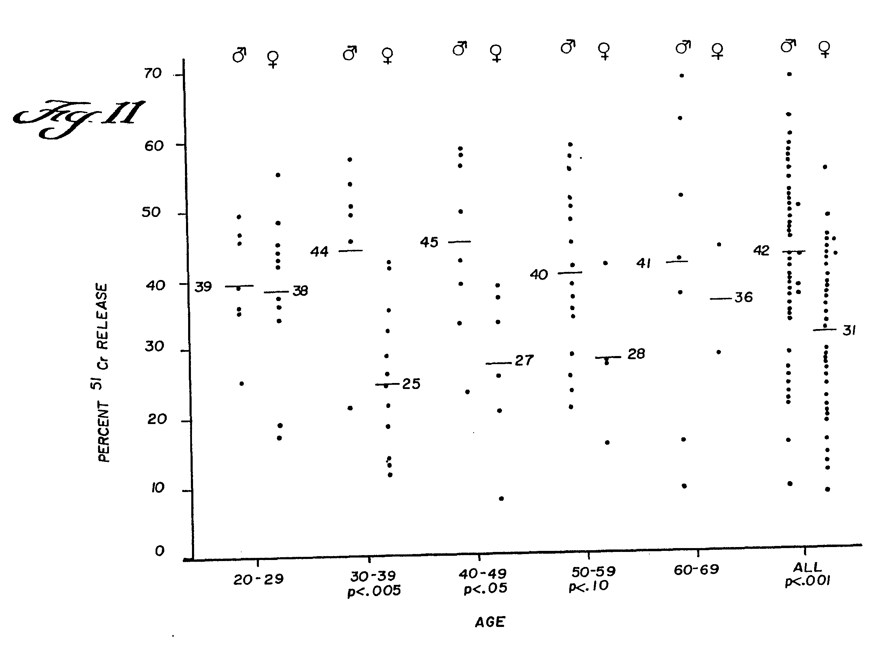

Figure 10 shows that males (41.6% killing) have a significantly higher NK cell activity ($p< .001$) than females (30.5% killing). This difference is also statistically significant for patients with a low family history of cancer ($p< .005$, Figure 10).

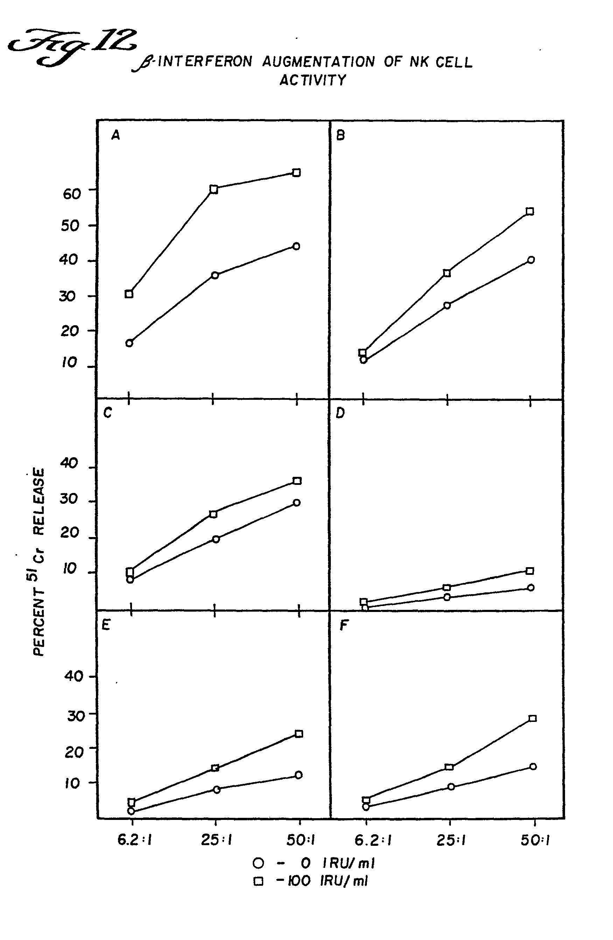

Figure 11 shows that the difference in NK cell activity does not result from age distribution differences between males and females since there is no age decade in which NK activity in females is greater than NK activity in males.

The NK cells results presented in all Figures are averages of four separate assays (4 and 5 hours, both at effector:target cell ratios of 25:1 and 50:1), each done in quadruplicate. A priori it was not possible to predict which incubation times and effector:target cell ratios would optimize the detection of NK cell defects among members of the study group. Accordingly, in studies of NK cell activity we routinely included three incubation times (3, 4 and 5 hours) each at three different effector:target cell ratios (6.2:1, 25:1 and 50:1). Since each of these nine assays was done in quadruplicate, 36 separate assays were performed in each individual.

### C. Failure of natural or synthetic IFN alone to correct the defect in cancer-predisposed people

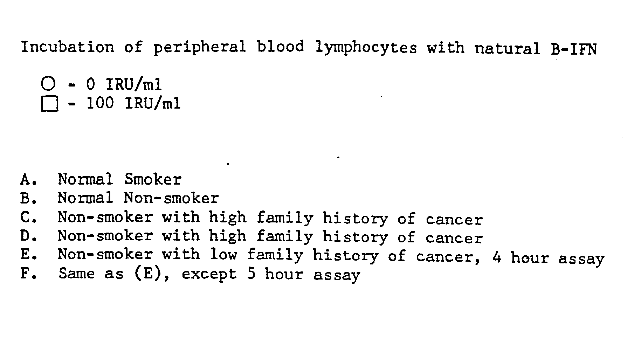

Figure 12 shows that natural fibroblast interferon (2000 units/ml, β-IFN) augments NK cell activity of individuals with elevated (panel A), normal (panel B), or low (panels C, D, E and F) NK cell activity. Moreover, individuals vary in their NK augmentation response to fibroblast interferon. For example, panel A shows that NK activity in a normal smoker was boosted from moderately elevated levels to extremely high levels. Panel B shows that NK activity in a normal nonsmoker was augmented from normal levels to moderately elevated levels. In panels C and D two non-smokers with high family histories of cancer differed in their NK response to fibroblast interferon. Moderately low NK activity (panel C) was stimulated to normal levels in one of the individuals. In contrast, extremely depressed NK activity (panel D) was augmented only minimally in the other. In panels E and F, low NK activity discovered in a non-smoker with a high family history of cancer showed moderate boosting in both the 4 hour (panel E) and 5 hour (panel F) assays. Similar results were obtained with the cloned subtypes of alpha (leukocyte) interferon, as prepared by the method of Lee et al. (Can. Res., *42*, 1312, 1982) or the various natural types of alpha and immune (gamma) interferon as described by Claeys et al. (Brit. J. Haem., *50*, 85, 1982). Importantly the defects could not be corrected by any form of interferon when applied alone, even at high concentrations.

The data for Figure 12 were gathered by incubating peripheral blood lymphocytes with (100 IRU/ml) or without natural β-IFN for 60 minutes. After washing, NK cell activity was assayed. Comparable results were obtained with alpha (leukocyte) and gamma (immune) IFN, both the natural variety and those made by recombinant DNA technology. In all instances, the defects were only partially corrected by IFN alone.

D. Complete correction of the NK defects
By $rI_n \cdot r(C_{12},U)_n$ and mismatched dsRNAs
Mismatched dsRNAs were prepared and tested in the above systems as described by Zarling et al (J. Immunol., *124*, 1852, 1980).

In all instances (see panels C, D, E, F at Figure 4) the defects were fully corrected such that the NK cells from patients at risk of developing cancer could no longer be distinguished from normal individuals (panels A and B, Figure 12).

That Ampligen and IFN form a synergistic combination can further be seen from the data in Table 5B.

TABLE 5B

Growth (% of control) of human bladder cancer cells in vitro in presence of various human Interferons alone and in combination with Ampligen: The synergistic effect of mixtures of Ampligen and Interferon.

|  | Without Ampligen | With Ampligen (200 Hg/ml) | Observed synergistic effect of Ampligen |
| --- | --- | --- | --- |
| Interferon alpha (200 IRU/ml) | 89 | 30 | 2.53 fold |
| Interferon clone A (250 IRU/ml) | 98 | 44 | 2.23 fold |
| Interferon beta (20 IRU/ml) | 50 | 17 | 2.94 fold |
| Interferon clone beta-serine (150 IRU/ml) | 63 | 36 | 1.75 fold |
| Interferon gamma (10 IRU/ml) | 95 | 0 (complete tumor cell) | 9.5 fold |

Kill

The column in Table 5B headed "Observed Synergistic Effect of Ampligen" shows the effect over and above the predicted additive effect of interferon plus ampligen. Ampligen alone resulted in a 14% decrease in tumor cell growth. Cells were grown in the standard manner in petri dishes with a 5% $CO_2$ atmosphere and all results were performed in triplicate. Interferons used were of various purities ($1 \times 10^5$ IRU/mg protein to $1 \times 10^8$ IRU/mg protein) and purified by hydrophobic affinity chromatography (Carter, *Pharmacology and Therapeutics,* Volume 8, pp 359—377, 1980) or by immobilized monoclonal antibodies, all techniques commonly practiced by those working in this art. The drug combinations were mixed together from their component parts and then added directly to the tumor cells.

Concurrently, tumor cells—before and after each of the treatments—were tested for the percent reduction in oncogene content. Oncogenes (abbreviated "onc") as noted elsewhere in this application, are specific nucleic acid sequences within human and animal chromosomes which, when activated, can cause uncontrolled cell growth—in short, malignancy itself. Oncogenes are imparted to living cells,.including human, by tumor-causing viruses and can be hereditarily transferred as well. Control of oncogenes thus provides a novel and unobvious means to arrest cancer at one of its earliest or initial steps.

Oncogenes measured in the experiment cited above consisted of those designated "Rous sarc" and "Harvey Ras" and their activity (expression) was monitored by the Quick-blot technique as described

17

(Bresser et al, *Proceedings National Academy of Sciences, U.S.,* in press, 1983). Oncogene probes were prepared in the standard manner and the assays conducted on nitrocellulose filter paper with NaI as described. Overall cellular DNA content served as a reference mark against which the % reduction in oncogene activity was measured.

Importantly, the combination of Ampligen with each of the interferons studied resulted in a synergistic inhibitory effect on oncogene expression in human bladder cancer cells. Similar effects will be observed with various other human cancer cells which possess different oncogenes such as those designated as follows: "v-ras-Harvey", "c-ras-Harvey", "v-ras-Kirsten", "v-abl", "c-myc", "v-sis", "c-mos", "v-sac-Rous", etc.

Ampligen treatment affords a therapeutic opportunity to patients when their tumors become resistant to interferon. A typical medical case study is as follows: a middle-aged white male (initials F.Z.) was diagnosed as having colon cancer and a fresh tumor specimen obtained for analysis in the clonogenic assay (reference cited in Legend to Table 5C). Prior to starting interferon therapy, 63% of his tumor cells died on exposure to interferon beta (500 IRU/ml), whereas after 6.5 months of therapy, a repeat biopsy showed less than a 40% tumor cell kill in vitro with the same interferon concentration. Interestingly, the patient received only a very low daily dose of interferon (300,000 IRU) and his tumor cells still became resistant. (Human adults with cancer can receive up to 300 million IRU per day which also leads to the development of resistant tumor cells). Before, as well as after the course of interferon therapy, the patient's (F.Z) tumor cells remained sensitive to Ampligen, as 250 µg/ml consistantly killed over 56% of the tumor cells.

Ampligen treatment affords a therapeutic opportunity in patients whose tumor cells are de novo resistant to Interferon, as shown in Table 5C.

TABLE 5C

Antiproliferative (cell growth inhibitory) activity of $rI_n\text{-}r(C_{12},U)_n$ [Ampligen] against various fresh human tumors studied in a tumor cell clonogenic assay

| Sample # | Histologic type | % Change in tumor cell colonies |
|---|---|---|
| 1. | renal | 97.6% decrease |
| 2. | renal | 88.8% decrease |
| 3. | renal | 87.9% decrease |
| 4. | renal | 87.3% decrease |
| 5. | renal | 86.7% decrease |
| 6. | renal | 81.9% decrease |
| 7. | renal | 80.5% decrease |
| 8. | colon | 78.5% decrease |
| 9. | adenoca unknown origin | 72.4% decrease |
| 10. | renal | 71.8% decrease |
| 11. | renal | 68.1% decrease |
| 12. | melanoma | 62.2% decrease |
| 13. | ovarian | 58.9% decrease |
| 14. | melanoma | 57.8% decrease |
| 15. | carcinoid | 56.0% decrease |
| 16. | breast | 52.1% decrease |
| 17. | renal | 43.0% decrease |
| 18. | breast | 41.1% decrease |

# EP 0 113 162 B1

TABLE 5C  (contd.)

Antiproliferative (cell growth inhibitory) activity of $rI_n$-$r(C_{12},U)_n$ [Ampligen] against various fresh human tumors studied in a tumor cell clonogenic assay

| Sample # | Histologic type | % Change in tumor cell colonies |
|---|---|---|
| 19. | renal | 40.4% decrease |
| 20. | leiomyosarcoma | 37.8% decrease |
| 21. | endometrial stromal sarcoma | 34.1% decrease |
| 22. | neurofibrosarcoma | 29.3% decrease |
| 23. | carcinoid | 19.7% decrease |
| 24. | ovarian | 19.1% decrease |
| 25. | colon | 18.6% decrease |
| 26. | mesothelioma | 16.9% decrease |
| 27. | breast | 16.5% decrease |
| 28. | colon | 15.4% decrease |
| 29. | ovarian | 11.0% decrease |
| 30. | colon | 9.5% decrease |
| 31. | melanoma | 0.0% decrease |
| 32. | leiomysarcoma | 9.3% increase |

Legend: All studies were conducted at an Ampligen concentration of 250 µg/ml. Colony counts were determined on day 10—21 depending on the growth rate of cells. Methods are described in Salmon et al. (*Recent Results in Cancer Research,* volume 74, pp 300—305, 1980, and *New England Journal of Medicine,* Volume 298, pp. 1321—1327, 1978).

Table 5C shows that 50% of a representative sampling of human tumors are sensitive to Ampligen when the criterion of sensitivity is defined as a 50% or greater decrease in tumor cell colonies. Such a criterion is well recognized by those who practice this art and it is accepted that a 50% or greater reduction in tumor cells by this assay predicts a favorable clinical response to the test drug. Of the seven patients with renal (kidney) cancer, three were unresponsive to interferon alone. For example, patient 5 showed less than a 32% tumor cell decrease at a very high concentration of human beta interferon (2,400 IRU/ml) whereas his tumor demonstrated an 86.7% decrease on exposure to Ampligen.

A "mixture" as claimed includes the attachment of either component in the mixture to an inert carrier (e.g. interferon to albumin or ampligen to a polycationic compound such as polylysine) for modulating the component's in vivo activity, and also results in a synergistic effect.

## Claims

1. A therapeutic composition comprising a therapeutic mixture of a dsRNA and an interferon or dsRNA and hemin.

2. A composition as claimed in claim 1 wherein said dsRNA is $rI_n \cdot rC_n$.

3. A composition as claimed in Claim 2, wherein the interferon is a naturally occurring interferon and is human fibroblast interferon or human leukocyte interferon.

4. A composition as claimed in Claim 2, wherein the interferon is a hybrid form and is an interferon derived in part from an alpha interferon and in part from a beta or gamma interferon.

5. A composition as claimed in Claim 1, wherein said interferon occurs naturally, is derived by means of recombinant DNA technology, or is a hybrid form.

6. A composition as claimed in any one of Claims 1 to 4 wherein said dsRNA is mismatched.

7. A composition as claimed in Claim 6, wherein said mismatched dsRNA is $rI_n \cdot r(C_{12},U)_n$.

# EP 0 113 162 B1

8. A therapeutic composition of matter useful for the treatment of cancer comprising a therapeutically effective mixture of a dsRNA in combination with hemin.

9. The use of a combination of (1) a dsRNA and (2) an interferon of hemin in the preparation of a medicament for use in the treatment of cancer by therapy or prophylaxis.

10. The use claimed in Claim 9, wherein proliferation of the cells of said cancer is resistant to interferon in vitro.

11. The use claimed in Claim 10, wherein said cells are resistant to the interferon of the medicament to the extent that less than a 50% kill of said cells is achieved.

12. The use of a dsRNA in the manufacture of a medicament to be used in combination with an interferon or hemin in the treatment of cancer by therapy or prophylaxis.

13. The use of an interferon in the manufacture of a medicament to be used in combination with a dsRNA in the treatment of cancer by therapy or prophylaxis.

14. The use of hemin in the manufacture of a medicament to be used in combination with a dsRNA in the treatment of cancer by therapy or prophylaxis.

## Patentansprüche

1. Eine therapeutische Zusammensetzung mit einem Gehalt an einer therapeutischen Mischung aus einer dsRNA und einem Interferon oder dsRNA und Hämin.

2. Eine Zusammensetzung nach Anspruch 1, dadurch gekennzeichnet, daß die dsRNA $rI_n \cdot rC_n$ ist.

3. Eine Zusammensetzung nach Anspruch 2, dadurch gekennzeichnet, daß das Interferon ein natürlich auftretendes Interferon ist und humanes Fibroblasten-Interferon oder humanes Leukocyten-Interferon ist.

4. Eine Zusammensetzung nach Anspruch 2, dadurch gekennzeichnet, daß das Interferon in einer Hybridform vorliegt und ein Interferon ist, das teilweise aus einem alpha-Interferon und teilweise aus einem beta- oder gamma-Interferon stammt.

5. Eine Zusammensetzung nach Anspruch 1, dadurch gekennzeichnet, daß das Interferon natürlich auftritt, mit DNA-Rekombinationsverfahren hergestellt wurde oder eine Hybridform ist.

6. Eine Zusammensetzung nach jeglichem der Ansprüche 1 bis 4, dadurch gekennzeichnet, daß die dsRNA fehlgepaart ist.

7. Eine Zusammensetzung nach Anspruch 6, dadurch gekennzeichnet, daß die fehlgepaarte dsRNA $rI_n \cdot r(C_{12},U)_n$ ist.

8. Eine therapeutische Stoffzusammensetzung, welche zur Behandlung von Krebs geeignet ist, gekennzeichnet durch eine therapeutisch wirksame Mischung einer dsRNA in Kombination mit Hämin.

9. Verwendung einer Kombination aus (1) einer dsRNA und (2) eines Interferons oder Hämins zur Herstellung eines Arzneimittels zur Verwendung bei der Behandlung von Krebs durch Therapie oder Prophylaxe.

10. Verwendung nach Anspruch 9, dadurch gekennzeichnet, daß die Proliferation der Krebszellen in vitro Interferon-resistent ist.

11. Verwendung nach Anspruch 10, dadurch gekennzeichnet, daß die Zellen gegenüber dem in dem Medikament enthaltenen Interferon in einem solchen Ausmaß resistent sind, daß weniger als 50% der Zellen abgetötet werden.

12. Verwendung einer dsRNA bei der Herstellung eines Arzneimittels zur Verwendung in Kombination mit einem Interferon oder Hämin bei der Behandlung von Krebs durch Therapie oder Prophylaxe.

13. Verwendung eines Interferons xei der Herstellung eines Arzneimittels zur Verwendung in Kombination mit einer dsRNA bei der Behandlung von Krebs durch Therapie oder Prophylaxe.

14. Verwendung von Hämin bei der Herstellung eines Arzneimittels zur Verwendung in Kombination mit einer dsRNA bei der Behandlung von Krebs durch Therapie oder Prophylaxe.

## Revendications

1. Composition thérapeutique comprenant un mélange thérapeutique d'un ARNds et d'un interféron ou d'un ARNds et d'hémine.

2. Composition selon la revendication 1, dans laquelle ledit ARNds est un $rI_n \cdot rC_n$.

3. Composition selon la revendication 2, dans laquelle l'interféron est un interféron d'origine naturelle et est l'interféron de fibroblastes humains ou l'interféron de leucocytes humains.

4. Composition selon la revendication 2, dans laquelle l'interféron est une forme hybride et est un interféron dérivé en partie d'un interféron α et en partie d'un interféron β ou γ.

5. Composition selon la revendication 1, dans laquelle ledit interféron est d'origine naturelle, est dérivé au moyen de la technologie de l'ADN recombinant ou est une forme hybride.

6. Composition selon l'une quelconque des revendications 1 à 4, dans laquelle l'ARNds est désapparié.

7. Composition selon la revendication 6, dans laquelle ledit ARNds désapparié est un $rI_n \cdot r(C_{12},U)_n$.

8. Composition thérapeutique de matière utile pour le traitement du cancer, comprenant un mélange thérapeutiquement efficace d'un ARNds en combinaison avec de l'hémine.

9. Utilisation d'une combinaison de (1) un ARNds et (2) un interféron ou de l'hémine dans la préparation d'un médicament utilisable dans le traitement thérapeutique ou prophylactique du cancer.

20

10. Utilisation selon la revendication 9, dans laquelle la prolifération des cellules dudit cancer est résistante à l'interféron in vitro.

11. Utilisation selon la revendication 10, dans laquelle lesdites cellules sont résistantes à l'interféron du médicament dans la mesure où il est obtenu une destruction de moins de 50%, desdites cellules.

12. Utilisation d'un ARNds dans la fabrication d'un médicament destiné à être utilisé en combinaison avec un interféron ou de l'hémine dans le traitement thérapeutique ou prophylactique du cancer.

13. Utilisation d'un interféron dans la fabrication d'un médicament destiné à être utilisé en combinaison avec un ARNds dans le traitement thérapeutique ou prophylactique du cancer.

14. Utilisation d'hémine dans la fabrication d'un médicament destiné à être utilisé en combinaison avec un ARNds dans le traitement thérapeutique ou prophylactique du cancer.

Fig. 2

Fig. 1

1

A.  Growth of HT1080-IFN$^r$ cells in HuIFN-B

    ● - 0 I.U./ml
    △ - 1000 I.U./ml after culture for 4 weeks in IFN-free
        medium
    ▲ - 1000 I.U./ml after culture for 15 weeks in IFN-free
        medium

B.  Growth of HT1080-IFN$^r$ cells in rIn·rCn

    ● - 0 mM
    □ - 0.1 mM
    ▲ - 0.2 mM
    △ - 0.5 mM
    ○ - 2.5 mM

C.  Growth of RT4-IFN$^r$ cells in HuIFN-B

    ● - 0 I.U./ml
    △ - 100 I.U./ml after culture for 0 weeks in IFN-free
        medium
    ▲ - 100 I.U./ml after culture for 4 weeks in IFN-free
        medium

D.  Growth of RT4-IFN$^r$ cells in rIn·rCn

    ● - 0 μM
    ■ - 0.1 μM
    □ - 5.0 μM
    ○ - 10.0 μM

FIGURE 2

HOURS POST TREATMENT

*Fig. 3*

A.  IFN$^S$ HT1080-Cl2 cells grown in
    presence of IFN

    ● - 0 U/ml
    □ - 90 U/ml
    ▲ - 100 U/ml
    ○ - 200 U/ml
    ■ - 400 U/ml


B.  IFN$^S$ HT1080-Cl2 cells grown in
    presence of $rI_n \cdot rC_n$

    ● - 0 mM
    □ - 0.1 mM
    ▲ - 0.2 mM
    ○ - 0.5 mM


C.  IFN$^r$ Ht1080-Cl2 cells grown in
    presence of rIn·rCn

    ● - 0 mM
    □ - 0.1 mM
    ▲ - 0.2 mM
    ○ - 0.5 mM
    ■ - 1.0 mM
    △ - 2.5 mM


D.  IFN$^S$ RT4-Cll cells grown in
    presence of IFN

    ● - 0 U/ml
    □ - 20 U/ml
    ▲ - 40 U/ml
    ○ - 80 U/ml
    △ - 160 U/ml


E.  IFN$^S$ RT4-Cll cells grown in
    presence of $rI_n \cdot rC_n$

    ● - 0 μM
    □ - 0.1 μM
    ■ - 5 μM
    ○ - 10 μM


F.  IFN$^r$ RT4-Cll cells grown in
    presence of $rI_n$-rCn

    ● - 0 μM
    □ - 0.1 μM
    ■ - 5 μM
    ○ - 10 μM


FIGURE 3

4

*Fig. 4*

| | | | 10mg↓ | | 15mg↓ | | |
|---|---|---|---|---|---|---|---|
| DAY | -2 | 0 | 1 | 4 | 5 | 6 | 7 |
| MATURE GRANULOCYTES | 3 | 5 | 15 | 14 | 26 | 25 | 36 |
| METAMYELOCYTES | 1 | 2 | 4 | 3 | 16 | 11 | 10 |
| MYELOCYTES | 3 | 5 | 4 | 42 | 14 | 28 | 9 |
| TOTAL GRANULOCYTES | 7 | 12 | 23 | 59 | 56 | 64 | 55 |
| BLASTS | 57 | 41 | 22 | 18 | 11 | 16 | 16 |

*Fig. 5*

*Fig. 6*

| | 10mg↓ | | 15mg↓ | | | | 30mg↓ | | 30mg↓ | | |
|---|---|---|---|---|---|---|---|---|---|---|---|
| DAY | 0 | 1 | 4 | 5 | 6 | 7 | 8 | 11 | 12 | 14 | 25 |
| MATURE GRANULOCYTES | 0 | 0 | 0 | 1 | 1 | 2 | 7 | 10 | 28 | 30 | 34 |
| META-MYELOCYTES | 0 | 0 | 2 | 2 | 2 | 3 | 5 | 8 | 10 | 5 | 2 |
| MYELOCYTES | 0 | 0 | 0 | 2 | 4 | 2 | 20 | 13 | 8 | 6 | 3 |
| TOTAL GRANULOCYTES | 0 | 0 | 2 | 5 | 7 | 7 | 32 | 31 | 46 | 41 | 39 |
| BLASTS | 47 | 44 | 67 | 75 | 69 | 77 | 48 | 62 | 41 | 29 | 13 |

## FIG. 7

## FIG. 8

EP 0 113 162 B1

*Fig. 9*

8

Fig. 10

Fig. 11

EP 0 113 162 B1

*Fig. 12* β-INTERFERON AUGMENTATION OF NK CELL ACTIVITY

O — 0 IRU/ml
□ —100 IRU/ml

11

Incubation of peripheral blood lymphocytes with natural B-IFN

○ - 0 IRU/ml
☐ - 100 IRU/ml

A. Normal Smoker
B. Normal Non-smoker
C. Non-smoker with high family history of cancer
D. Non-smoker with high family history of cancer
E. Non-smoker with low family history of cancer, 4 hour assay
F. Same as (E), except 5 hour assay